(19) **Europäisches Patentamt**

**European Patent Office**

**Office européen des brevets**

(11) **EP 1 086 705 A1**

(12) **EUROPEAN PATENT APPLICATION**

published in accordance with Art. 158(3) EPC

(43) Date of publication:
28.03.2001 Bulletin 2001/13

(51) Int. Cl.[7]: **A61K 45/06**, A61K 39/395
// (A61K31/35, 31:40, 31:44)

(21) Application number: 99921205.3

(22) Date of filing: 20.05.1999

(86) International application number:
PCT/JP99/02660

(87) International publication number:
WO 99/59636 (25.11.1999 Gazette 1999/47)

(84) Designated Contracting States:
AT BE CH CY DE DK ES FI FR GB GR IE IT LI LU
MC NL PT SE

(30) Priority: 20.05.1998 JP 13899998

(71) Applicant:
KYOWA HAKKO KOGYO CO., LTD.
Chiyoda-ku, Tokyo 100-8185 (JP)

(72) Inventors:
• SHITARA, Kenya
Kanagawa 251-0025 (JP)
• SATO, Yasufumi
Sendai-shi, Miyagi 989-3201 (JP)

(74) Representative:
Kinzebach, Werner, Dr. et al
Patentanwälte
Reitstötter, Kinzebach und Partner
Postfach 86 06 49
81633 München (DE)

(54) **VEGF ACTIVITY INHIBITORS**

(57) The present invention provides a therapeutic agent which is effective for solid tumors, arthritis in chronic rheumatoid arthritis, diabetic retinopathy, retinopathy of prematurity, psoriasis, or the like, comprising a combination of a substance which inhibits signal transduction mediated by human VEGF receptor Flt-1 which is useful for the diagnosis or treatment of diseases in which their morbid states progress by abnormal angiogenesis, such as proliferation or metastasis of solid tumors, arthritis in chronic rheumatoid arthritis, diabetic retinopathy, retinopathy of prematurity, psoriasis, and the like with a substance which inhibits signal transduction mediated by human VEGF receptor KDR.

EP 1 086 705 A1

Printed by Xerox (UK) Business Services
2.16.7 (HRS)/3.6

**Description**

<u>Technical Field</u>

The present invention relates to a medicament comprising a combination of a substance which inhibits signal transduction mediated by human VEGF receptor Flt-1 with a substance which inhibits signal transduction mediated by human VEGF receptor KDR and is useful for treatment of diseases in which their morbid states progress by abnormal angiogenesis, such as proliferation or metastasis of solid tumors, arthritis in rheumatoid arthritis, diabetic retinopathy, retinopathy of prematurity, psoriasis, and the like.

<u>Background Art</u>

Angiogenesis plays an important role in formation of a circulatory system and construction of various tissues at fetus in vertebrates, is directly involved in the formation of the corpus luteum during the sexual cycle, transient proliferation of the uterine endometrium and formation of the placenta in mature individuals (females). With regard to pathological states, angiogenesis is involved in the proliferation or metastasis of solid tumors and formation or acceleration of morbidity in diabetic retinopathy and rheumatoid arthritis [*J. Biol. Chem*., *267*: 10931 (1992)]. Angiogenesis occurs by the secretion of an angiogenesis factor and involves the process of a tube formation and producing a new blood vessel. During this process, the basement membrane and interstitum are destroyed by a protease secreted from endothelial cells of an existing blood vessel around the secreted angiogenesis factor, followed by subsequent migration and proliferation of vascular endothelial cells [*J. Biol. Chem*., *267*: 10931 (1992)]. Factors which induce angiogenesis include vascular permeability factor (hereinafter referred to as "VPF") and vascular endothelial growth factor (hereinafter referred to as "VEGF") (hereinafter referred to as "VPF/VEGF"). These factors are considered the most important factors in pathological and non-pathological angiogenesis [*Advances in Cancer Research, 67*: 281 (1995)]. VPF/VEGF is a protein having a molecular weight of about 40,000 constituted by homodimers, which had been reported to be independent molecules as vascular permeability factor (VPF) in 1983 [*Science, 219*: 983 (1983)] and as vascular endothelial growth factor (VEGF) in 1989 [*Biochem. Biophys. Res. Comm., 161*: 851 (1989)], but it has been revealed as the results of cDNA cloning that they are the same substance [*Science, 246*: 1306 (1989); *Science, 246*: 1309 (1989)] (hereinafter, the term "VPF/VEGF" is referred to as "VEGF"). Beyond the activity of VEGF upon vascular endothelial cells described above, VEGF has also been shown to have a growth enhancing activity [*Biochem. Biophys. Res. Comm., 161*: 851 (1989)], a migration enhancing activity [*J. Immunology, 152*: 4149 (1994)], a metalloprotease secretion enhancing activity [*J. Cell Physiol., 153*: 557 (1992)], a urokinase and tPA secretion enhancing activity [*Biochem. Biophys. Res. Comm., 181*: 902 (1991)], and the like. Furthermore, VEGF has been shown to have an angiogenesis enhancing activity [*Circulation, 92* suppl II: 365 (1995)], a vascular permeability enhancing activity [*Science, 219*: 983 (1983)], and the like as its *in vivo* activities. It has been reported that VEGF is a growth factor having extremely high specificity for vascular endothelial cells [*Biochem. Biophys. Res. Comm., 161*: 851 (1989)] and that four proteins having different molecular weight are present due to alternative splicing of mRNA [*J. Biol. Chem., 267*: 26031 (1991)].

Among diseases accompanied by angiogenesis, it has been reported that VEGF plays an important role in the proliferation or metastasis of solid tumors and formation of morbid states of diabetic retinopathy and rheumatoid arthritis. With regard to solid tumors, production of VEGF in a number of human tumor tissues has been reported, such as in renal carcinoma [*Cancer Research, 54*: 4233 (1994)], breast cancer [*Human Pathology, 26*: 86 (1995)], brain tumor [*J. Clinical Investigation, 91*: 153 (1993)], gastrointestinal cancer [*Cancer Research, 53*: 4727 (1993)], ovarian cancer [*Cancer Research, 54*: 276 (1994)], and the like. Also, results of a study on the correlation between VEGF expression quantity in tumors and survival ratio of patients in patients with breast cancer have revealed that tumor angiogenesis is more active in tumors expressing high levels of VEGF than low VEGF expression tumors and that the survival ratio is lower in breast cancer patients having high VEGF expression tumors than breast cancer patients having low VEGF expression tumors [*Japanese J. Cancer Research, 85*: 1045 (1994)]. It has been reported also that an anti-VEGF monoclonal antibody inhibited tumor growth in a xenograft model test system in which a human tumor was transferred into nude mice by subcutaneous transplantation [*Nature, 362*: 841 (1993)]. Also, it has been reported that, in a metastatic cancer model of a human tumor in nude mice, an anti-VEGF monoclonal antibody inhibited metastasis of the tumor [*Cancer Research, 56*: 921 (1996)]. Additionally, since a high concentration of VEGF was detected in human carcinomatous pleural perfusions and ascites, the possibility that VEGF is a major factor involved in the retention of pleural perfusions and ascites has been suggested [*Biochimica et Biophysica Acta, 1221*: 211 (1994)], and inhibition of the retention of pleural perfusions and ascites is expected by blocking VEGF.

In diabetic retinopathy, abnormal angiogenesis causes retinal detachment and hemorrhage of the vitreous body, resulting in blindness, and it has been reported that angiogenesis in diabetic retinopathy and the expression level of VEGF in the patient's eye balls are positively correlative [*New England J. Medicine, 331*: 1480 (1994)]. Also, it has been reported that angiogenesis in a monkey retinopathy model is inhibited when the VEGF activity is inhibited, by the intraocular administration of an anti-VEGF neutralizing monoclonal antibody [*Arch. Ophthalmol., 114*: 66 (1996)].

Progress in the morbid states of rheumatoid arthritis (destruction of bone and cartilage) is accompanied by angiogenesis, and it has been reported that a high concentration of VEGF is contained in the synovial fluid of patients with rheumatoid arthritis and that macrophages in joints of patients with rheumatoid arthritis produce VEGF [*Journal of Immunology, 152*: 4149 (1994); *J. Experimental Medicine, 180*: 341 (1994)].

Two VEGF receptors have been reported, which are Flt-1 (fms-like tyrosine kinase) that is the first receptor belonging to the receptor-type tyrosine kinase family [*Oncogene, 5*: 519 (1990); *Science, 255*: 989 (1992)] and KDR (kinase insert domain-containing receptor) that is the second receptor [WO 92/14748; *Biochem. Biophys. Res. Comm., 187*: 1579 (1992)]. A mouse type homologue of human type VEGF receptor KDR is called Flk-1 [*Proc. Natl. Acad. Sci. USA, 88*: 9026 (1991), WO 94/11499, *Cell, 72*: 835 (1993)]. The extracellular domain of Flt-1 and KDR/Flk-1 is a membrane protein of 180 to 200 kilodalton in molecular weight which having 7 immunoglobulin-like regions and an intracellular domain consisting of a tyrosine kinase region. It has been reported that VEGF specifically binds to Flt-1 and KDR/Flk-1 at Kd values of 20 pM and 75 pM and that Flt-1 and KDR/Flk-1 are expressed in vascular endothelial cells in a specific manner [*Proc. Natl. Acad. Sci. USA, 90*: 7533 (1993); *Proc. Natl. Acad. Sci. USA, 90*: 8915 (1993)].

With regard to Flt-1 in various diseases, it has been reported that, in comparison with vascular endothelial cells in normal tissues, expression of flt-1 mRNA increases in tumor vascular endothelial cells of human glioblastoma tissues [*Nature, 359*: 845 (1992)] and tumor vascular endothelial cells of human digestive organ cancer tissues [*Cancer Research, 53*: 4727 (1993)]. Additionally, it has been reported that expression of flt-1 mRNA is observed by *in situ* hybridization in vascular endothelial cells of joints of patients with rheumatoid arthritis [*J. Experimental Medicine, 180*: 341 (1994)]. These results strongly suggest that a VEGF/VEGF receptor Flt-1 system plays an important role in tumor angiogenesis. Although it has been reported that VEGF binds to Flt-1 and the intracellular domain is auto-phosphorylated [*Science, 255*: 989 (1992)], the detailed function of the receptor mechanism is still unclear. However, it has been discovered that knock out mice in which the flt-1 gene was destroyed die after a fetal age of 8.5 to 9.5 days due to abnormal blood vessel construction caused by abnormal morphology of vascular endothelial cells during blood island formation in the early stage of development and subsequent angiogenesis. This had led to an assumption that Flt-1 has a function essential for the tube formation of vascular endothelial cells in angiogenesis [*Nature, 376*: 66 (1995)].

On the other hand, regarding the expression of KDR in various human diseases, it has been reported that the expression of KDR at the mRNA level is increased in tumor vascular endothelial cells of human brain tumor tissues [*American J. Pathology, 146*: 368 (1995)] and tumor vascular endothelial cells of human gastrointestinal cancer tissues [*Cancer Research, 53*: 4727 (1993)] in comparison with the vascular endothelial cells of normal tissues. These results strongly suggest that the VEGF-VEGF receptor KDR system is taking an important role in the tumor angiogenesis. In addition, it has been reported that expression of KDR mRNA by *in situ* hybridization is also found in joint vascular endothelial cells of rheumatoid arthritis patients [*J. Experimental Medicine, 180*: 341 (1994)], thus indicating importance of the VEGF-VEGF receptor KDR system. Regarding functions of the VEGF receptor KDR/Flk-1, it has been reported that, among various activities of VEGF, KDR is concerned in the proliferation of vascular endothelial cells, because when KDR is expressed in swine artery endothelial cells, it reacts with VEGF to cause proliferation and migration [*J. Biol. Chem., 269*: 26988 (1994)]. Also, it has been reported, that the KDR/Flk-1 relates to the proliferation and differentiation of vascular endothelial cells of an animal individual because mature vascular endothelial cells were not found in a flk-1 knockout mouse prepared by destroying the mouse flk-1 gene, and its blood island of yolk sac was not formed and died in the womb [*Nature, 376*: 62 (1995)].

As discussed above, among various functions of VEGF, it is assumed that proliferation of vascular endothelial cells depends on KDR, and organization of vasculture depends on Flt-1, but it is not known about which one of these receptors is responsible in other activities of VEGF, such as mediating acceleration of vascular permeability, promotion of protease production, and the like. Since the angiogenesis abnormalities found in flt-1 knockout mouse and KDR/flk-1 knockout mouse are completely different from each other, it is assumed that angiogenesis could be inhibited effectively when these two receptors are simultaneously blocked.

It has been reported that anti-KDR/Flk-1 ribozyme and anti-Flt-1 ribozyme capable of inhibiting expression of KDR/Flk-1 and Flt-1 in vascular endothelial cells can inhibit VEGF-dependent proliferation of human skin microtubule vascular endothelial cells HMVEC, but as a partial inhibition in both cases, and stronger growth inhibition effect was observed when expression of the two receptors was simultaneously inhibited by simultaneously adding the anti-KDR/Flk-1 ribozyme and anti-Flt-1 ribozyme (WO 97/15662).

Based on the above, it is expected that a method in which various biological activities of VEGF are inhibited by inhibiting functions of KDR and Flt-1 using two anti-VEGF receptors, KDR and Flt-1, monoclonal antibodies in combination would be useful in treating diseases in human in which their morbid states progress by abnormal angiogenesis, such as proliferation or metastasis of solid tumors, arthritis in rheumatoid arthritis, diabetic retinopathy, retinopathy of prematurity, psoriasis and the like. However, there are no reports on the effectiveness of the use of two anti-VEGF receptors, KDR and Flt-1, monoclonal antibodies in combination.

Disclosure of the Invention

Methods which are useful in treating diseases in which their morbid states progress by abnormal angiogenesis, such as proliferation or metastasis of solid tumors, arthritis in rheumatoid arthritis, diabetic retinopathy, retinopathy of prematurity, psoriasis, and the like, are desired. Although there is a report on an anti-VEGF receptor KDR monoclonal antibody (Subject No. A-52, Angiogenesis and Cancer, AACR Special Conference in Cancer Research, January 25, 1998), it cannot completely inhibit various activities of VEGF, even if it can inhibit only KDR. Accordingly, the development of a medicament which can inhibit various biological activities of VEGF effectively has been desired.

The present invention relates to the following (1) to (14).

(1) A medicament comprising a combination of a substance which inhibits signal transduction mediated by human VEGF receptor Flt-1 with a substance which inhibits signal transduction mediated by human VEGF receptor KDR.
     The substance which inhibits signal transduction mediated by a receptor includes a substance which inhibits binding of a ligand to the receptor, a substance which inhibits signal transduction from the receptor, and the like.
     The medicament comprising the combination may be either a medicament comprising a substance capable of inhibiting signal transduction mediated by human VEGF receptor Flt-1 and a substance which inhibits signal transduction mediated by human VEGF receptor KDR, or a medicament which uses a substance which inhibits signal transduction mediated by human VEGF receptor Flt-1 and a substance which inhibits signal transduction mediated by human VEGF receptor KDR simultaneously at the time of administration.
     The substance which inhibits signal transduction mediated by human VEGF receptor Flt-1 may be any substance which has an activity of inhibiting signal transduction mediated by human VEGF receptor Flt-1, and examples include an anti-human VEGF receptor Flt-1 monoclonal antibody having a neutralization activity, p38 inhibitor, such as SB203580 [*Oncogene, 15*: 2169 (1997)] *etc*., and the like.
     The substance which inhibits signal transduction mediated by human VEGF receptor KDR way be any substance having an activity of inhibiting signal transduction mediated by human VEGF receptor KDR, and examples include an anti-human VEGF receptor KDR monoclonal antibody having a neutralization activity, KDR tyrosine kinase inhibitor, such as SU5416 [*Cancer Research, 59*: 99 (1999)] *etc*., a medicament which inhibits ERK by MEK 1 inhibition, such as PD98059 [*Journal of Biological Chemistry, 270*: 27489 (1995)] *etc*., and the like.

(2) A VEGF activity inhibitor comprising a combination of a substance which inhibits signal transduction mediated by human VEGF receptor Flt-1 with a substance which inhibits signal transduction mediated by human VEGF receptor KDR.

(3) An angiogenesis inhibitor comprising a combination of a substance which inhibits signal transduction mediated by human VEGF receptor Flt-1 with a substance which inhibits signal transduction mediated by human VEGF receptor KDR.

(4) A therapeutic agent for a disease in which the morbid states progress by abnormal angiogenesis, comprising a combination of a substance which inhibits signal transduction mediated by human VEGF receptor Flt-1 with a substance which inhibits signal transduction mediated by human VEGF receptor KDR.

(5) The therapeutic agent according to the above (4), wherein the disease in which the morbid states progress by abnormal angiogenesis is proliferation or metastasis of a solid tumor, arthritis in rheumatoid arthritis, diabetic retinopathy, retinopathy of prematurity, or psoriasis.

(6) An agent according to the above (1) to (5), wherein the substance which inhibits signal transduction mediated by human VEGF receptor Flt-1 is a substance which inhibits binding of VEGF to the Flt-1 receptor or a substance which inhibits signal transduction from Flt-1 receptor.

(7) The agent according to the above (6), wherein the substance which inhibit, binding of VEGF to the Flt-1 receptor is selected from an anti-human VEGF receptor Flt-1 monoclonal antibody and a fragment of the antibody.

(8) The agent according to the above (7), wherein the anti-human VEGF receptor Flt-1 monoclonal antibody is a monoclonal antibody belonging to the mouse IgG2b subclass produced by a hybridoma KM1750 (FERM BP-5700) or a monoclonal antibody belonging to the mouse IgG1 subclass produced by a hybridoma KM1732 (FERM BP-5698).

(9) The agent according to the above (6), wherein the substance which inhibits signal transduction from Flt-1 receptor is selected from a substance having Flt-1 tyrosine kinase inhibition activity and a substance having p38 inhibition activity.

(10) An agent according to the above (1) to (5), wherein the substance which inhibits signal transduction mediated by human VEGF receptor KDR is a substance which inhibits binding of VEGF to the KDR receptor or a substance which inhibits signal transduction from the KDR receptor.

(11) The agent according to the above (10), wherein the substance which inhibits binding of VEGF to the KDR receptor is selected from an anti-human VEGF receptor KDR monoclonal antibody and a fragment of the antibody.

(12) The agent according to the above (11), wherein the anti-human VEGF receptor KDR monoclonal antibody is a monoclonal antibody belonging to the mouse IgG1 subclass produced by a hybridoma KM1992 (FERM BP-6217) or a monoclonal antibody belonging to the mouse IgG2b subclass produced by a hybridoma KM1995 (FERM BP-6218).

(13) The agent according to the above (10), wherein the substance which inhibits signal transduction from KDR receptor is selected from a substance having KDR tyrosine kinase inhibition activity and a substance having ERK inhibition activity.

(14) A medicament comprising a human VEGF receptor Flt-1 antagonist and a human VEGF receptor KDR antagonist.

The receptor antagonist means a substance which inhibits functions of a receptor, and it can be any one of low molecular or high molecular substances, so long as they can inhibit the functions of a receptor. Examples include a substance which inhibits binding of a ligand to the receptor, preferably neutralizing antibodies, and a substance which inhibits signal transduction mediated by a receptor (hereinafter also referred to as "signal inhibition").

The present inventors found that the biological activity of VEGF which is inhibited by an anti-VEGF receptor KDR monoclonal antibody and the biological activity of VEGF which is inhibited by an anti-VEGF receptor Flt-1 monoclonal antibody are different from each other and further found that various biological activities of VEGF can be effectively inhibited by inhibiting functions of KDR and Flt-1 using the anti-VEGF receptors, KDR and Flt-1, monoclonal antibodies in combination and that, as an unexpected effect, synergistic activities on the inhibition of some biological activities of VEGF can be obtained when the two anti-VEGF receptors, KDR and Flt-1, monoclonal antibodies are used in combination. Accordingly, when a substance which inhibits signal transduction mediated by human VEGF receptor Flt-1 and a substance which inhibits signal transduction mediated by human VEGF receptor KDR are used in combination, treatment of the above diseases in which their morbid states progress by abnormal angiogenesis, such as proliferation or metastasis of solid tumors, arthritis in rheumatoid arthritis, diabetic retinopathy, retinopathy of prematurity, psoriasis, and the like, can be carried out more effectively.

The substances used in the present invention are not limited, so long as they are a substance which inhibits signal transduction mediated by human VEGF receptor Flt-1 (hereinafter simply referred to as "Flt-1") and a substance which inhibits signal transduction mediated by human VEGF receptor KDR (hereinafter simply referred to as "KDR").

The substance which inhibits signal transduction mediated by Flt-1 can be any substance, so long as it can inhibit function of Flt-1. Examples include an anti-Flt-1 monoclonal antibody, a fragment of the antibody and soluble Flt-1, which inhibit binding of VEGF to Flt-1, a tyrosine kinase inhibitor which inhibits signal transduction of Flt-1, p38 inhibitor, such as SB203580 [*Oncogene, 15*: 2169 (1997)], and the like.

The substance which inhibits signal transduction mediated by KDR can be any substance, so long as it can inhibit functions of KDR. Examples include an anti-KDR monoclonal antibody, a fragment of the antibody and soluble KDR, which inhibit binding of VEGF to KDR, a tyrosine kinase inhibitor which inhibits the signal transduction of KDR, such as SU5416 [*Cancer Research, 59*: 99 (1999)] *etc*., an inhibition of MEK1 [abbreviation of MAP (abbreviation of "mitogen-activated protein") kinase] which is an ERK (abbreviation of "extracellular signal-regulated protein kinase") activator, such as PD98059 [*Journal of Biological Chemistry, 270*: 27489 (1995)] *etc*., and the like .

Examples of the monoclonal antibody include an antibody produced by a hybridoma and a recombinant antibody produced by a transformant transformed with an expression vector containing the antibody gene.

The recombinant antibody includes a humanized antibody, an antibody fragment, such as a single chain antibody, a disulfide stabilized antibody *etc*., and the like, which are produced by genetic recombination. A recombinant antibody having characteristics of monoclonal antibody, low antigenicity and prolonged blood half-life is preferably used.

The humanized antibody used in the present invention includes a human chimeric antibody and a human CDR-grafted antibody.

The antibody fragment used in the present invention includes Fab (abbreviation of Fragment of antigen binding), Fab', F(ab')$_2$, a single chain antibody (single chain Fv; hereinafter referred to as "scFv") and a disulfide stabilized antibody (disulfide stabilized Fv; hereinafter referred to as "dsFv"), as antibody fragments which specifically react with Flt-1 or KDR.

Also, the antibody fragment includes a peptide selected from amino acid sequences of the complementary determining region (hereinafter referred to as "CDR") of antibody variable region (hereinafter also referred to as "V region") heavy chain (hereinafter also referred to as "H chain") (hereinafter, the antibody variable region heavy chain will also be referred to as "VH") and antibody V region light chain (hereinafter also referred to as "L chain") (hereinafter, the antibody variable region light chain will also be referred to as "VL") of the above antibody.

The human chimeric antibody means an antibody comprising an antibody variable region heavy chain and variable region light chain of a non-human animal antibody, a constant region heavy chain (hereinafter referred to as "CH") of a human antibody, and a constant region light chain (hereinafter referred to as "CL") of a human antibody.

The human chimeric antibody used in the present invention can be produced by obtaining cDNA encoding VH and VL from a hybridoma capable of producing a monoclonal antibody which specifically reacts with Flt-1 or KDR, inserting them into an expression vector for animal cell having a gene encoding a human antibody CH and a human antibody CL to construct a human chimeric antibody expression vector, and then expressing the antibody by introducing the vector into an animal cell.

The structure of the human chimeric antibody used in the present invention may belong to any immunoglobulin (Ig) class, but the C region of an IgG type immunoglobulin, more preferably IgG1, IgG2, IgG3, IgG4 or the like belonging to the IgG type, is preferred.

The human CDR-grafted antibody means an antibody in which CDRs of VH and VL of a human antibody are replaced with respective CDR sequences of a non-human animal antibody.

The human CDR-grafted antibody used in the present invention can be produced by constructing cDNA encoding V regions in which any CDR sequences of VH and VL of a human antibody are replaced with corresponding CDR sequences of VH and VL of a non-human animal antibody, which specifically reacts with Flt-1 or KDR, inserting them into an expression vector for animal cell having a gene encoding human antibody CH and human antibody CL to construct a human CDR-grafted antibody expression vector, and then expressing the antibody by introducing the vector into an animal cell.

The structure of the human CDR-grafted antibody C region used in the present invention may belong to any immunoglobulin (Ig) class, but the C region of an immunoglobulin of IgG type, more preferably IgG1, IgG2, IgG3, IgG4 or the like class belonging to the IgG type, is preferred.

The Fab is a fragment having a molecular weight of about 50,000 and antigen-binding activity which comprises about half of the N-terminal side of H chain and a full portion of L chain obtained by digesting, with an enzyme, papain, the peptide moiety of the upper side of two disulfide bonds that cross-link two H chains at the hinge region of IgG.

The Fab used in the present invention can be obtained by treating an anti-human VEGF receptor Flt-1 antibody with papain. Alternatively, the Fab can be produced by inserting a DNA fragment which encodes the Fab fragment of the antibody into an expression vector for animal cells, and introducing the vector into an animal cell to express the antibody of interest.

The Fab' is a fragment of about 50,000 in molecular weight having antigen-binding activity, and it is obtained by cleaving the disulfide bond between hinges of the above-described F(ab')$_2$.

The Fab' used in the present invention can be obtained by treating an anti-human VEGF receptor Flt-1 antibody with a reducing agent, dithiothreitol. Alternatively, the Fab' can be produced by inserting a DNA fragment which encodes the Fab' fragment of the antibody into an expression vector for animal cells, and introducing the vector into an animal cell to express the antibody of interest.

The F(ab')$_2$ is a fragment having a molecular weight of about 100,000 and antigen-binding activity, comprising two Fab regions bonded at the hinge region, which is obtained by digesting, with an enzyme, trypsin, the lower side of two disulfide bonds at the hinge region of IgG.

The F(ab')$_2$ used in the present invention can be obtained by digesting an anti-human VEGF receptor Flt-1 antibody with trypsin. Alternatively, F(ab')$_2$ can be produced by inserting a DNA fragment which encodes the F(ab')$_2$ fragment of the antibody into an expression vector for animal cells, and introducing the vector into an animal cell to express the antibody of interest.

The single chain antibody (scFv) means a VH-P-VL or VL-P-VH polypeptide obtained by linking a VH chain with a VL chain using an appropriate peptide linker (hereinafter referred to as "L"). The VH and VL of the scFv of the present invention can be any of the monoclonal antibody and human CDR-grafted antibody of the present invention.

The single chain antibody used in the present invention can be produced by isolating cDNAs encoding VH and VL from a hybridoma capable of producing an anti-human VEGF receptor Flt-1 antibody to construct a single chain antibody expression vector, inserting the cDNAs into the scFv expression vector, and introducing the expression vector into *Escherichia coli*, yeast or an animal cell to express the antibody of interest.

The disulfide stabilized antibody (dsFv) means an antibody obtained by bonding, via a disulfide bond, polypeptides in which one amino acid residue in each of VH and VL is replaced with a cysteine residue. The amino acid residue to be replaced with a cysteine residue can be selected based on the three-dimensional structure estimation of antibodies in accordance with the method reported by Reiter *et al*. [*Protein Engineering, 7*: 697 (1994)]. The VH or VL contained in the disulfide stabilized antibody of the present invention can be any of the monoclonal antibody and human CDR-grafted antibody of the present invention.

The disulfide stabilized antibody used in the present invention can be produced by isolating cDNAs encoding VH and VL from a hybridoma capable of producing an anti-human VEGF receptor Flt-1 antibody, inserting the cDNAs into an appropriate expression vector, introducing the expression vector into *Escherichia coli*, yeast or an animal cell to express the antibody of interest.

Examples of the anti-Flt-1 monoclonal antibody include a monoclonal antibody KM1732 belonging to the mouse IgG1 subclass produced by a hybridoma KM1732 (FERM BP-5698) and a monoclonal antibody KM1750 belonging to the mouse IgG2b subclass produced by a hybridoma KM1750 (FERM BP-5700). Also, examples of the KDR monoclonal antibody include a monoclonal antibody KM1992 belonging to the mouse IgG1 subclass produced by a hybridoma KM1992 (FERM BP-6217) and a monoclonal antibody KM1995 belonging to the mouse IgG2b subclass produced by a hybridoma KM1995 (FERM BP-6218). The hybridomas KM1732 and KM1750 have been deposited on October 8, 1996, in National Institute of Bioscience and Human Technology, Agency of Industrial Science and Technology, as FERM BP-5698 and FERM BP-5700, respectively. The hybridomas KM1992 and KM1995 have been deposited on January 8, 1998, in National Institute of Bioscience and Human Technology, Agency of Industrial Science and Technology, as FERM BP-6217 and FERM BP-6218, respectively.

The methods for the production of the anti-KDR antibody as a substance which inhibits signal transmission mediated by KDR and the anti-Flt-1 antibody as a substance which inhibits signal transmission mediated by Flt-1, that constitute the present invention, and use of medicaments containing these substances are explained below.

1. Production methods of anti-human VEGF receptor KDR monoclonal antibody and anti-human VEGF receptor Flt-1 antibody

(1) Preparation of antigen

Examples of the substance useful as the antigen for the production of the anti-human VEGF receptor KDR monoclonal antibody and the anti-human VEGF receptor Flt-1 antibody include cells in which human VEGF receptor KDR protein and human VEGF receptor Flt-1 protein are expressed on the cell surface or a cell membrane fraction thereof, soluble human VEGF receptor KDR protein and soluble human VEGF receptor Flt-1 protein having an extracellular region of different length, a fusion protein of the protein with Fc region of the antibody, and the like.

Examples of the cells capable of expressing human VEGF receptor KDR and human VEGF receptor Flt-1 on the cell surface include NIH3T3-KDR cells and NIH3T3-Flt-1 cells [*Cell Growth & Differentiation, 7*: 213 (1996)]. As a method for expressing as a soluble human VEGF receptor KDR protein and soluble human VEGF receptor Flt-1 protein having an extracellular region of different length or a fusion protein of the protein with Fc region of the antibody, the full length or a partial fragment of cDNA which encodes human VEGF receptor KDR and human VEGF receptor Flt-1 [*Cell Growth & Differentiation, 7*: 213 (1996)] [*Oncogene, 5*: 519 (1990)] is inserted into a downstream site of the promoter of an appropriate vector, the thus constructed recombinant vector is inserted into host cells and the thus obtained human VEGF receptor KDR and human VEGF receptor Flt-1 expression cells are cultured in an appropriate medium to produce the full length of a partial fragment of human VEGF receptor KDR and human VEGF receptor Flt-1 in the cells or culture supernatant as such or as a fusion protein.

The hosts can be any one of bacteria, yeast, animal cells, insect cells and the like so long as they can express the gene of interest. Examples of the bacteria include the genus *Escherichia*, the genus *Bacillus* and the like, such as *Escherichia coli, Bacillus subtilis* and the like. Examples of the yeast include *Saccharomyces cerevisiae, Schizosaccharomyces pompe*, and the like. Examples of the animal cells include namalwa cells which are human cells, COS cells which are monkey cells, CHO cells which are Chinese hamster cells, and the like. Examples of the insect cells include Sf9 and Sf21 (manufactured by Pharmingen), High Five (manufactured by In Vitrogen), and the like.

The vector to which the DNA of the present invention is inserted can be any vector so long as the DNA can be inserted and expressed in a host cell.

When a bacterium such as *Escherichia coli* is used as the host, the expression vector can be preferably constructed with a promoter, a ribosome binding sequence, the DNA of the present invention, a transcription termination sequence and, if necessary, a promoter controlling sequence. Examples include commercially available pGEX (manufactured by Pharmacia), pET System (manufactured by Novagen), and the like.

With regard to the method for introducing the recombinant vector into a bacterium, any one of the known methods for introducing DNA into bacteria, such as a method in which calcium ion is used [*Proc. Natl. Acad. Sci. USA, 69*: 2110 (1972)], a protoplast method (Japanese Published Unexamined Patent Application No. 248394/91), and the like can be used.

When yeast is used as the host, YEp13 (ATCC 37115), YEp24 (ATCC 37051), YCp50 (ATCC 37419), or the like is used as the expression vector.

With regard to the method for introducing the recombinant vector into yeast, any one of the known methods for introducing DNA into yeast, such as an electroporation method [*Methods, Enzymol., 194*: 182 (1990)], a spheroplast method [*Proc. Natl. Acad. Sci. USA, 84*: 1929 (1978)], a lithium acetate method [*J. Bacterial., 153*: 163 (1983)], and the like can be used.

When animal cells are used as the host, pAGE107 [Japanese Published Unexamined Patent Application No. 22979/88; *Cytotachnology, 3*: 133 (1990)], pAGE103 [*J. Biochem., 101*: 1307 (1987)], and the like can be exemplified as the useful expression vector.

Any promoter can be used so long as it can be expressed in animal cells. Examples include the promoter of IE (immediate early) gene of cytomegalovirus (CMV), the SV40 promoter, the metallothionein promoter and the like.

Furthermore, the enhancer of the IE gene of human CMV may be used together with the promoter.

With regard to the method for the introduction of the recombinant vector into animal cells, any one of the known methods for introducing DNA into animal cells, such as an electroporation method [*Cytotechnaogy, 3*: 133 (1990)], a calcium phosphate method (Japanese Published Unexamined Patent Application No. 227075/90), a lipofection method [*Proc. Natl. Acad. Sci. USA, 84*: 7413 (1987)] and the like can be used.

When insect cells are used as the host, the protein can be expressed by the known method described in, for example, *Current Protocols*, Supplement 1-34 and *Baculovirus Expression Vectors, A Laboratory Manual*. That is, the recombinant gene introducing vector and baculovirus described in the following are simultaneously introduced into insect cells to obtain a recombinant virus in the insect cell culture supernatant and then the insect cells are infected with the thus obtained recombinant virus to obtain protein-expressing insect cells.

Examples of the gene introducing vector include pVL1392, pVL1393, pBlueBacIII (all manufactured by In Vitrogen), and the like.

Examples of the baculovirus include Autograph californica nuclear polyhedrosis virus with which insects of the family *Barathra* are infected.

With regard to the method for the simultaneous introduction of the above-described recombinant gene introducing vector and the above-described baculovirus into insect cells for the production of the recombinant virus, calcium phosphate method (Japanese Published Unexamined Patent Application No. 227075/90), lipofection method [*Proc. Natl. Acad. Sci. USA, 84*: 7413 (1987)] and the like can be exemplified.

Alternatively, the protein of interest can be produced by producing a recombinant baculovirus with, for example, BaculoGold Starter Kit manufactured by Pharmigen and then infecting the above-described insect cells, such as Sf9, Sf21, High Five, or the like, with the recombinant virus [*Bio/Technology, 10*: 457 (1988)].

With regard to the gene expression method, techniques, such as secretion production, fusion protein expression and the like have been developed, and each of these methods can be used. For example, it can be carried out in accordance with the method described in *Molecular Cloning*, 2nd edition, Cold Spring Harbor Lab. Press, New York (1989).

The full length or a partial fragment of human VEGF receptor KDR and human VEGF receptor Flt-1 can be produced as such or as a fusion protein thereof by culturing a transformant obtained in the above-described manner in a culture medium to form and accumulate the protein of the present invention in the resulting culture mixture, and then recovering the protein from the culture mixture.

Culturing of the transformant of the present invention in a culture medium is carried out in accordance with a usual method which is used in the culturing of respective hosts.

With regard to the medium for use in the culturing of the transformant obtained using a microorganism, such as *Escherichia coli*, yeast, or the like, as the host, either a natural medium or a synthetic medium can be used, so long as it contains materials which can be assimilated by the microorganism, such as carbon sources, nitrogen sources, inorganic salts, and the like, and can perform culturing of the transformant efficiently [*Molecular Cloning*, 2nd edition, Cold Spring Harbor Lab. Press, New York (1989)]. The culturing is carried out generally under aerobic conditions, such as a shaking culture, submerged agitation aeration culture, or the like, at 15 to 40°C for 16 to 96 hours. During the culturing, the pH is controlled to 3.0 to 9.0. Adjustment of the pH is carried out using an inorganic or organic acid, an alkali solution, urea, calcium carbonate, ammonia, and the like. During the culturing, if necessary, antibiotics, such as ampicillin, tetracycline, and the like may be added to the medium.

With regard to the medium for use in the culturing of a transformant obtained using animal cells as the host, RPMI 1640 medium, Eagle's MEM medium or any one of these media further supplemented with fetal calf serum may be used. The culturing is carried out generally at 35 to 37°C for 3 to 7 days in the presence of 5% $CO_2$. During the culturing, if necessary, antibiotics, such as kanamycin, penicillin, and the like may be added to the medium.

With regard to the medium for use in the culturing of a transformant obtained using insect cells as the host, TNM-FH medium (manufactured by Pharmingen), Sf900IISFM (manufactured by Life Technologies), ExCell400 or ExCell405 (both manufactured by JRH Biosciences), or the like may be used. The culturing is carried out generally at 25 to 30°C for 1 to 4 days, and during the culturing, if necessary, antibiotics, such as gentamicin and the like, can be added to the medium.

Although media for the culturing of animal cells and insect cells contain serum, it is desirable to use a serum-free medium in order to efficiently purify the full length or a partial fragment of human VEGF receptor KDR and human VEGF receptor Flt-1 as such or as a fusion protein.

When the full length or a partial fragment of human VEGF receptor KDR and human VEGF receptor Flt-1 is accumulated inside the host cells as such or as a fusion protein, the cells after completion of the culturing are collected by centrifugation, suspended in an aqueous buffer and then disrupted using ultrasonic oscillator, French press, or the like, and subsequently recovering the protein from a supernatant fluid produced by centrifuging the thus disrupted cells.

Also, when an insoluble body is formed inside the cells, the insoluble body is solubilized using a protein denaturing agent and then higher-order structure of the protein is formed by diluting or dialyzing the thus solubiiized protein in or against a solution which does not contain the protein denaturing agent or contains the agent but in such a low concentration that the protein is not denatured.

When the full length or a partial fragment of human VEGF receptor KDR and human VEGF receptor Flt-1 is secreted outside the cells as such or as a fusion protein, the expressed protein can be collected from the culture supernatant. The isolation and purification can be carried out by employing separation means, such as solvent extraction, fractional precipitation with organic solvents, salting out, dialysis, centrifugation, ultracentrifugation, ion exchange chromatography, gel filtration chromatography, hydrophobic chromatography, affinity chromatography, reverse phase chromatography, crystallization, electrophoresis, and the like, alone or in combination.

(2) Immunization of animals and preparation of antibody-producing cells

Although any one of animals, such as mice, rats, hamsters, rabbits, and the like, can be used in the immunization, so long as a hybridoma can be produced, an example in which mice and rats are used is described in the present invention. A mouse or rat of 3 to 20 weeks of age is immunized with the protein obtained in the above 1(1) as the antigen, and antibody-producing cells are collected from the spleen, lymph node or peripheral blood of the animal. The immunization is carried out by administering the antigen several times through subcutaneous, intravenous or intraperitoneal injection together with an appropriate adjuvant. As the adjuvant, a complete Freund's adjuvant or a combination of aluminum hydroxide gel with pertussis vaccine can be exemplified. A blood sample is collected from the fundus of the eye or caudal vein of the animal 3 to 7 days after each administration, the sample is tested, for example, by enzyme immunoassay [*Enzyme-linked Immunosorbent Assay (ELISA)*, published by Igaku Shoin (1976)] as to whether it is reactive with the antigen used, namely soluble human VEGF receptor KDR and soluble human VEGF receptor Flt-1 or NIH3T3 cells in which human VEGF receptor KDR and human VEGF receptor Flt-1 are expressed on the cell surface, and then a mouse or rat showing sufficient antibody titer in their sera is submitted for use as the supply source of antibody-producing cells. On the 3rd to 7th day after final administration of the antigen, the spleen is excised from the immunized mouse or rat to carry out fusion of the spleen cells with myeloma cells in accordance with the known method [*Antibodies - A Laboratory Manual*, Cold Spring Harbor Laboratory (1988); hereinafter referred to as "*Antibodies - A Laboratory Manual*"].

(3) Preparation of myeloma cells

As the myeloma cells, any myeloma cells capable of growing *in vitro* may be used, which include established cells obtained from mouse, such as 8-azaguanine-resistant mouse (BALB/c) myeloma cell line P3-X63Ag8-U1 (P3-U1) [G. Kohler *et al., Europ. J. Immunol, 6*: 511 (1976)], SP2/O-Ag14 (SP-2) [M. Shulman *et al., Nature, 276*: 269 (1978)], P3-X63-Ag8653 (653) [J.F. Kearney *et al., J. Immunol., 123*: 1548 (1979)], P3-X63-Ag8 (X63) [*G. Kohler et al., Nature, 256*: 495 (1975)], and the like. These cell lines are cultured and subcultured in accordance with the known method (*Antibodies - A Laboratory Manual*) and $2\times10^7$ or more of the cells are secured until cell fusion.

(4) Cell fusion

The antibody-producing cells obtained in (2) and the myeloma cells obtained in (3) are washed, respectively, and mixed with cell aggregating medium, polyethylene glycol-1000 (PEG-1000) or the like, to carry out cell fusion and then suspended in a culture medium. For washing of the cells, MEM medium or PBS (1.83 g of disodium hydrogen phosphate, 0.21 g of potassium dihydrogen phosphate, 7.65 g of sodium chloride, 1 liter of distilled water, pH 7.2) is used. In order to obtain the fused cells of interest selectively, HAT medium {normal medium [a medium prepared by adding glutamine (1.5 mM), 2-mercaptoethanol ($5\times10^{-5}$ M), gentamicin (10 µg/ml) and fetal calf serum (FCS) (10%, manufactured by CSL) to RPMI-1640 medium] further supplemented with hypoxanthine ($10^{-4}$ M), thymidine ($1.5\times10^{-5}$ M) and aminopterin ($4\times10^{-7}$ M)} is used as the medium for suspending the fused cells.

After the culturing, a portion of the culture supernatant is sampled and a hole specifically reacting human VEGF receptor KDR and human VEGF receptor Flt-1 or a recombinant protein of human VEGF receptor KDR and human VEGF receptor Flt-1 described in (1) or the like is selected by the enzyme immunoassay described in (5). Thereafter, cloning is carried out twice by limiting dilution analysis [HT medium (a medium from which aminopterin has been removed is firstly used, and a normal medium is secondly used], and a hybridoma which shows stably high antibody titer is selected as a hybridoma capable of producing an anti-human VEGF receptor KDR monoclonal antibody and a hybridoma capable of producing an anti-human VEGF receptor Flt-1 monoclonal antibody.

(5) Selection of anti-human VEGF receptor KDR monoclonal antibody and anti-human VEGF receptor Flt-1 monoclonal antibody

Selection of a hybridoma capable of producing an anti-human VEGF receptor KDR monoclonal antibody and a hybridoma capable of producing an anti-human VEGF receptor Flt-1 monoclonal antibody is carried out by enzyme immunoassay described below.

Enzyme immunoassay

An antigen protein, cells which expresses an antigen protein or the like is coated on a 96-well plate, and the reaction is carried out using a hybridoma culture supernatant or a purified antibody obtained in the above method as a first antibody.

After the reaction of the first antibody, the plate is washed and a second antibody is added.

The second antibody is obtained by labeling an antibody which can recognize immunoglobulin of the first antibody or anti-rat immunoglobulin antibody with biotin, an enzyme, a chemiluminescent substance, a radioactive compound or the like. Specifically, if a mouse is used for the production of the hybridoma, an antibody which can recognize mouse immunoglobulin is used as a second antibody.

After the reaction, a reaction suitable for the substance used for labeling the second antibody is carried out in order to select a hybridoma producing a monoclonal antibody which specifically reacts with the antigen.

(6) Preparation of monoclonal antibody

The anti-human VEGF receptor Flt-1 monoclonal antibody-producing hybridoma cells and the anti-human VEGF receptor KDR monoclonal antibody-producing hybridoma obtained in 1(4) are administered by intraperitoneal injection into 8-to 10-week-old mice or nude mice treated with pristane [by intraperitoneal administration of 0.5 ml of 2,6,10,14-tetramethylpentadecane (pristane) followed by 2 weeks of feeding] at a dose of $2\times10^7$ to $5\times10^6$ cells/animal. The hybridoma causes ascites tumor in 10 to 21 days. The ascitic fluid is collected from the mice or nude mice, centrifuged, subjected to salting out with 40 to 50% saturated ammonium sulfate or to caprylic acid precipitation and then passed through a DEAE-Sepharose column, protein A column or Cellulofine GSL 2000 (manufactured by Seikagaku Kogyo) to collect an IgG or IgM fraction to give a purified monoclonal antibody.

The subclass of the purified monoclonal antibody can be determined using a mouse monoclonal antibody typing kit or a rat monoclonal antibody typing kit. The amount of protein can be determined by the Lowry method or by calculation based on the optical density at 280 nm.

The subclass of antibody means isotypes within the class, such as IgG1, IgG2a, IgG2b and IgG3 in the case of mouse, and IgG1, IgG2, IgG3 and IgG4 in the case of human.

The mouse IgG1 and IgG2a and human IgG1 types have complement-dependent cytotoxicity activity (hereinafter as "CDC activity") and antibody-dependent cellular cytotoxicity activity (hereinafter as "ADCC activity"), so that they are useful in applying to medical treatments.

Methods for inhibiting various biological activities of VEGF by inhibiting functions of human VEGF receptors KDR and Flt-1 through a combination of two anti-human VEGF receptors, KDR and Flt-1, monoclonal antibodies.

Examples of the method for measuring biological activities of VEGF include a VEGF-dependent proliferation test and migration test of vascular endothelial cells, a tubule formation test in *New Biochemical Experiment*, Course 10, Blood Vessels (Endothelium and Smooth Muscle) (Tokyo Kagaku Dojin, 1991), and the like.

Examples of the method for analyzing genes whose expression fluctuates accompanied by the activation of vascular endothelial cells due to VEGF stimulation include Northern blotting analysis, RT-PCR in *New Cell Engineering Experiment Protocol, Cell Engineerin*g supplement 8 (Shujunsha, 1993), an *in situ* hybridization method in *In Situ Hybridization Techniques* (Gakusai Kikaku, 1992), and the like.

Examples of the gene whose expression fluctuates accompanied by the activation of vascular endothelial cells due to VEGF stimulation include ets-1 [*J. Cellular Physiology*, *169*: 522 (1996)], MMP-1 [*J. Cellular Physiology, 169*: 522 (1996)], flt-1 [*Cancer Research, 57*: 5421 (1997)], and the like.

Examples of the method for analyzing proteins whose expression fluctuates accompanied by the activation of vascular endothelial cells due to VEGF stimulation include an immunoprecipitation method, Western blotting analysis, an immunocyte staining method in *Monoclonal Antibody Experiment Manual* (Kodansha Scientific, 1987), *Second Series Biochemical Experiment Course, Methods for Immunological Biochemistry Research* (Tokyo Kagaku Dojin, 1986), a receptor auto-phosphorylation measuring method in *Second Series Biochemical Experiment Course, Signal Transduction and Cell Response* (Tokyo Kagaku Dojin, 1986), and the like.

Examples of the protein whose expression fluctuates accompanied by the activation of vascular endothelial cells due to VEGF stimulation include p38 [*Oncogene, 15*: 2169 (1997)], ERK1 and ERK2 [*Oncogene, 15*: 2169 (1997)], JNK1 and JNK2 [*Oncogene, 15*: 2169 (1997)], and the like.

2. Use of medicament comprising a substance which inhibits signal transduction mediated by Flt-1 and a substance which inhibits signal transduction mediated by KDR

The medicament of the present invention comprising a substance which inhibits signal transduction mediated by Flt-1 and a substance which inhibits signal transduction mediated by KDR include a medicament comprising anti-human VEGF receptor Flt-1 monoclonal antibody and an anti-human VEGF receptor KDR monoclonal antibody, a medicament comprising an anti-human VEGF receptor Flt-1 monoclonal antibody fragment and an anti-human VEGF receptor KDR monoclonal antibody fragment, a medicament comprising an Flt-1 signal inhibitor and a KDR signal inhibitor, and the like. Also, these compositions may be a mixture of an antibody, antibody fragment, a chemical substance and the like, or may be a complex, such as a bispecific antibody in which two antibodies are bound and the like.

The medicament comprising the antibody of the present invention can be administered directly as a treating agent, but it is generally preferred to provide it in the form of a pharmaceutical medicament produced by mixing it with at least one pharmacologically acceptable carrier in accordance with optional methods well known in the technical field of pharmaceutics.

It is preferred to select a route of administration which is the most effective in carrying out the intended treatment, such as oral administration or parenteral administration that includes tracheal administration, rectal administration, subcutaneous injection, intramuscular injection, intravenous injection, and the like. Intravenous injection is preferred in the case of an antibody or peptide preparation.

The dosage form includes sprays, capsules, tablets, granules, syrups, emulsions, suppositories, injections, ointments, tapes, and the like.

Examples of the pharmaceutical preparation suitable for oral administration include emulsions, syrups, capsules, tablets, powders, granules, and the like.

Liquid preparations, such as emulsions and syrups, are produced using additives, such as water, saccharides (e.g., sucrose, sorbitol, fructose, *etc*.), glycols (e.g., polyethylene glycol, propylene glycol, *etc*.), oils (e.g., sesame oil, olive oil, soybean oil, *etc.*), antiseptics (e.g., p-hydroxybenzoic acid esters, *etc*.), flavors (e.g., strawberry flavor, peppermint, *etc*.), and the like.

Solid preparations, such as capsules, tablets, powders, granules, and the like, can be produced using additives, such as fillers (e.g., lactose, glucose, sucrose, mannitol, *etc*.), disintegrating agents (e.g., starch, sodium alginate, *etc*.), lubricating agents (e.g., magnesium stearate, *etc*.), binders (e.g., polyvinyl alcohol, hydroxypropylcellulose, gelatin, *etc*.), surfactants (e.g., fatty acid esters, *etc*.), plasticizers (e.g., glycerol, *etc*.), and the like.

Preparations suitable for parenteral administration include injections, suppositories, sprays, and the like.

Injections are prepared using a carrier or the like, such as a salt solution, glucose solution or a mixture thereof.

Suppositories are prepared using a carrier, such as cacao butter, hydrogenated fat, a carboxylic acid, or the like.

Sprays are prepared from the compound itself or using a carrier or the like which does not stimulate oral and airway mucous membranes of patients and can facilitate absorption of the compound by dispersing it as minute particles.

Examples of the carrier include lactose, glycerol, and the like. Depending on the properties of the antibody or peptide and the carrier to be used, other preparations, such as aerosols and dry powders, can be produced. The components exemplified as additives of oral preparations can also be added to these parenteral preparations.

Although a dose of the pharmaceutical composition of the present invention varies depending on the age, symptoms and the like of each patient, it is administered to mammals including human in a dose of 0.1 to 20 mg/kg/day as each monoclonal antibody. When the monoclonal antibodies are simultaneously administered, they are administered by intravenous injection once a day (single injection or everyday injection) or intermittently 1 to 3 times a week, once within 2 to 3 weeks, or when they are separately administered, each monoclonal antibody is administered by intravenous injection at an optional interval once a day (single injection or everyday injection) or intermittently 1 to 3 times a week, once within 2 to 3 weeks.

Use of the two monoclonal antibodies specific for VEGF receptors KDR and Flt-1 in combination shown by the present invention can be applied to the treatment of diseases caused by abnormal angiogenesis, efficiently and strongly by inhibiting various biological activities of VEGF and further showing synergistic effects.

Brief Description of the Drawings

Fig. 1 is a graph showing a result of the evaluation of single and combined use effects of anti-human VEGF receptor KDR monoclonal antibody and anti-human VEGF receptor Flt-1 monoclonal antibody on the VEGF-dependent human vascular endothelial cell HUVEC growth acceleration activity.

Fig. 2 is a graph showing a result of the evaluation of single and combined use effects of anti-human VEGF receptor KDR monoclonal antibody and anti-human VEGF receptor Flt-1 monoclonal antibody on the VEGF-dependent human vascular endothelial cell HUVEC migration acceleration activity.

Fig. 3 is a graph showing a result of the evaluation of the effect of anti-human VEGF receptor Flt-1 monoclonal antibody on the VEGF-dependent human vascular endothelial cell HUVEC migration acceleration activity.

Fig. 4 is a graph showing a result of the evaluation of single and combined use effects of anti-human VEGF receptor KDR monoclonal antibody and anti-human VEGF receptor Flt-1 monoclonal antibody on mRNA whose expression fluctuates by VEGF stimulation in human vascular endothelial cell HUVEC.

Fig. 5 is a graph showing a result of the evaluation of single and combined use effects of anti-human VEGF receptor KDR monoclonal antibody and anti-human VEGF receptor Flt-1 monoclonal antibody on p38, ERK1, ERK2, JNK1 and JNK2 whose expressions fluctuate by VEGF stimulation in human vascular endothelial cell HUVEC.

Fig. 6 is a graph showing a result of the evaluation of single and combined use effects of anti-human VEGF receptor KDR monoclonal antibody and anti-human VEGF receptor Flt-1 monoclonal antibody on human vascular endothelial cell HUVEC which becomes large by VEGF stimulation.

Fig. 7 is a graph showing construction steps of plasmid pVL1393/Flt 3N.

Fig. 8 is a graph showing construction steps of plasmid pVL1393/Flt 7N.

Fig. 9 is a graph showing SDS polyacrylamide gel electrophoresis (using a gel having a gradient of 5 to 20%) patterns of purified Flt-1 7N and Flt-1 3N. Starting from the left side, electrophoresis patterns of molecular marker, Flt-1 3N and Flt-1 7N are shown. The electrophoresis was carried out under reducing condition.

Fig. 10 is a graph showing a result of the analysis on the effect of soluble human VEGF receptors Flt-1 7N and Flt-1 3N to inhibit binding of $^{125}$I human VEGF to pre-coated soluble human VEGF receptors Flt-1 7N.

Fig. 11 is a graph showing construction steps of plasmid pVL-KDR-7N-Fc.

Fig. 12 is a schematic illustration showing various soluble KDR-Fc derivatives.

Fig. 13 is a schematic illustration showing various soluble KDR derivatives.

Fig. 14 is a graph showing SDS polyacrylamide gel electrophoresis (using a gel having a gradient of 5 to 20%) patterns of various purified soluble KDR-Fc derivatives. Starting from the left side, electrophoresis patterns of KDR-1N-Fc, KDR-2N-Fc, KDR-3N-Fc, KDR-4N-Fc, KDR-5N-Fc, KDR-7N-Fc, KDR-2Δ1N-Fc, KDR-4Δ1N-Fc and KDR-5Δ1N-Fc are shown. The electrophoresis was carried out under reducing condition.

Fig. 15A is a graph showing a result of the analysis on the effect of various soluble human VEGF receptor KDR-Fc derivatives to inhibit binding of $^{125}$I-human VEGF to pre-coated soluble human VEGF receptor KDR-7N-Fc. Fig. 15B is a graph showing a result of the analysis on the binding of $^{125}$I-human VEGF to various pre-coated soluble human VEGF receptor KDR-Fc derivatives.

Fig. 16 is a graph showing a result of the analysis on the binding activity of human VEGF receptor KDR monoclonal antibody to various soluble human VEGF receptor KDR-Fc derivatives.

Fig. 17 is a graph showing epitope regions of human VEGF receptor KDR monoclonal antibody.

Fig. 18 is a graph showing a result of the analysis on the binding activity of human VEGF receptor KDR monoclonal antibody to various soluble human VEGF receptor KDR-Fc derivatives.

Best Mode for Carrying Out the Invention

Example 1

Production of anti-human VEGF receptor Flt-1 monoclonal antibody and anti-human VEGF receptor KDR monoclonal antibody:

Each of hybridomas KM1732 (FERM BP-5698) and KM1750 (FERM BP-5700) capable of producing an anti-Flt-1 monoclonal antibody and hybridomas KM1992 (FERM BP-6217) and KM1995 (FERM BP-6218) capable of producing an anti-KDR monoclonal antibody was injected into the abdominal cavity of each pristane-treated female mouse (Balb/c) of 8 weeks of age at a dose of $5 \times 10^6$ to $20 \times 10^6$ cells/animal. The hybridoma caused ascites tumor in 10 to 21 days thereafter. The ascitic fluid was collected from the ascitic fluid-filled mice (1 to 8 ml/animal), centrifuged (3,000 rpm, 5 minutes) to remove solid matter and then purified by a caprylic acid precipitation method (Antibodies, A Laboratory Manual) to obtain a purified monoclonal antibody.

The subclass of the monoclonal antibody was determined by enzyme immunoassay using a subclass typing kit (manufactured by Zymed). As the result, KM1732 was a monoclonal antibody belonging to mouse IgG1 subclass, and KM1750 (FERM BP-5700) belonging to mouse IgG2b subclass, KM1992 (FERM BP-6217) belonging to mouse IgG1 subclass and KM1995 (FERM BP-6218) belonging to mouse IgG2b subclass.

Example 2

VEGF-dependent cell growth inhibition test using VEGF receptor monoclonal antibodies:

Effects of the two anti-VEGF receptors, KDR and Flt-1, monoclonal antibodies on the VEGF-dependent growth activity of human vascular endothelial cells was measured as an index of *in vitro* angiogenesis activity.

Human recombinant VEGF 165 protein was expressed and purified in accordance with the method of Cohen *et al*. [*Growth Factors, 7*: 133 (1992)] using a baculovirus-insect cell expression system.

Human umbilical vein endothelial cells (hereinafter referred to as "HUVEC"; manufactured by KURABO) were suspended in EBM medium (manufactured by Clonetics) containing 5% fetal calf serum (FCS) and EGM-ECGS (manufactured by Clonetics) and cultured on a type I collagen coat plate. The medium was changed to M-199 (manufactured by Nissui) medium containing 5% FCS 24 hours before the following test, followed by culturing.

The HUVEC was suspended in 200 $\mu$l of the medium to a density of $1 \times 10^4$ cells, inoculated into each well of a 96 well microtiter plate and cultured at 37°C in a $CO_2$ incubator until the cells became confluent. After the culturing, the cells were pre-cultured for 15 minutes by adding each anti-VEGF receptor monoclonal antibody (final concentration 0, 1 or 10 $\mu$g/ml) and then cultured for 24 hours by adding VEGF (final concentration 1 nM) and 1.0 $\mu$Ci [$^3$H]thymidine (manufactured by Amersham), and the [$^3$H]thymidine incorporated into DNA of the cells after their culturing was measured using liquid scintigraphy.

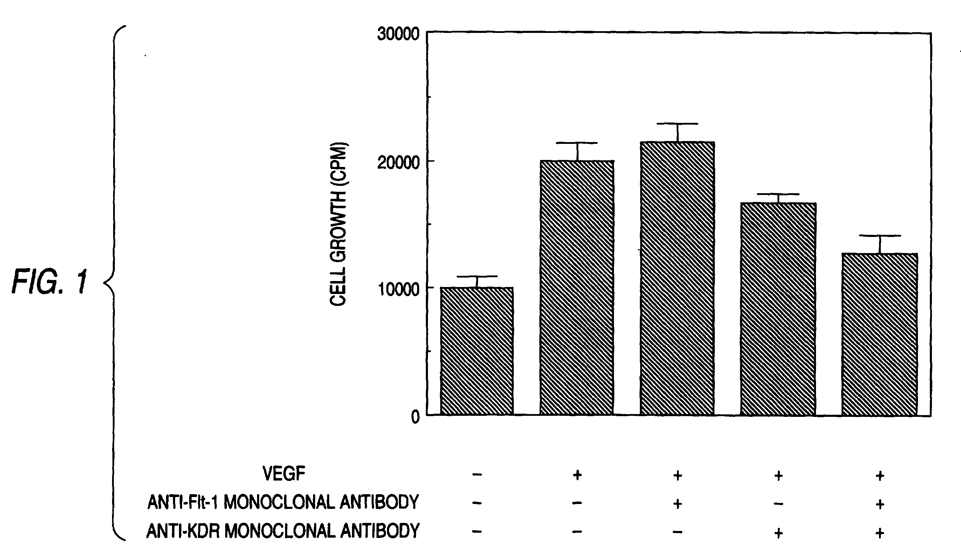

The results are shown in Fig. 1. Cell growth of HUVEC measured using incorporation of [$^3$H]thymidine as an index increased to about two times by the addition of VEGF, but the growth was not inhibited by the addition of anti-VEGF receptor Flt-1 monoclonal antibody KM1750 (final concentration 1 $\mu$g/ml). On the other hand, 30.7% of growth inhibition activity was observed by the addition of anti-VEGF receptor KDR monoclonal antibody KM1992 (final concentration 10 $\mu$g/ml). In addition, the inhibition activity was increased using anti-VEGF receptor Flt-1 monoclonal antibody KM1750 (final concentration 1 $\mu$g/ml) and anti-VEGF receptor KDR monoclonal antibody KM1992 (final concentration 10 $\mu$g/ml) in combination, and 69.3% of growth inhibition activity was observed. Thus, it was shown that KDR is a main receptor concerned in the growth of vascular endothelial cells and Flt-1 has a role in accelerating the growth activity mediated by KDR.

Based on the above, it was found that growth of vascular endothelial cells induced by VEGF is inhibited by the anti-KDR monoclonal antibody, and a synergistic growth inhibition effect is obtained by use in combination with the anti-Flt-1 monoclonal antibody.

Example 3

VEGF-dependent cell migration inhibition test using anti-VEGF receptor monoclonal antibodies:

Effects of the two anti-VEGF receptor, KDR and Flt-1, monoclonal antibodies on the VEGF-dependent migration activity of human vascular endothelial cells were evaluated as an index of *in vitro* angiogenesis activity.

The cell migration test was carried out in accordance with the method of Sato *et al*. [*J. Cell Biology*, *107*: 1199 (1988)]. The HUVEC cultured in a dish of 3.5 cm until it became confluent was scratched with a razor's edge and then washed with PBS. A 1.5 ml portion of M-199 medium containing 5% FCS was added and then VEGF (final concentration 10 ng/ml) and each anti-VEGF receptor monoclonal antibody (final concentration 0, 1 or 10 $\mu$g/ml) were added, and the cells were cultured for 24 hours. After the culturing, the number of cells wandered from the scratched position was measured.

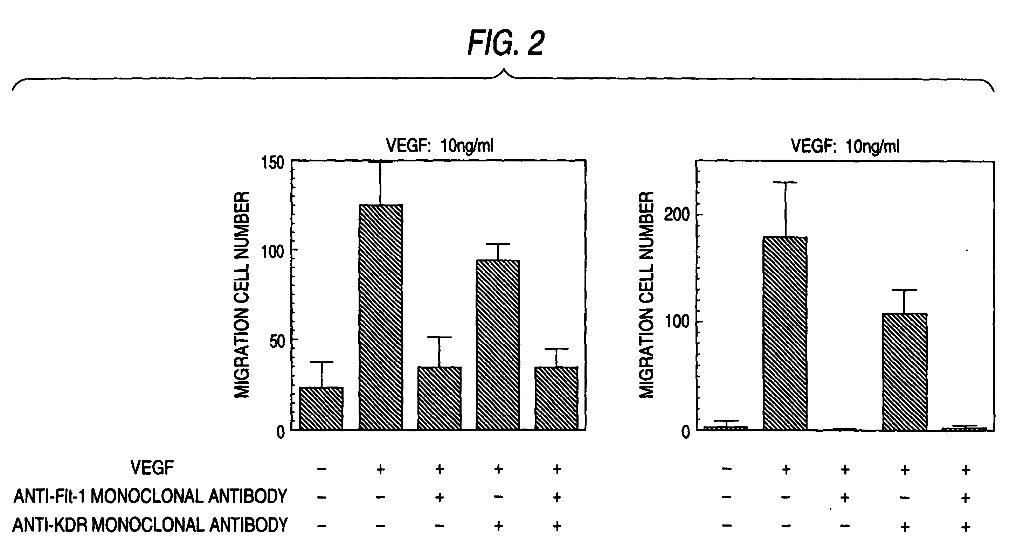

The results are shown in Fig. 2. Cell migration activity of HUVEC was increased by the addition of VEGF, but the migration was completely inhibited by anti-VEGF receptor Flt-1 monoclonal antibody KM1750 (final concentration 1 $\mu$g/ml). On the other hand, a partial weak migration inhibition activity was observed by anti-VEGF receptor KDR monoclonal antibody KM1992 (final concentration 10 $\mu$g/ml). Increase in the inhibition activity was not found when anti-VEGF receptor Flt-1 monoclonal antibody KM1750 (final concentration 1 $\mu$g/ml) and anti-VEGF receptor KDR monoclonal antibody KM1992 (final concentration 10 $\mu$g/ml) were used in combination. Thus, it was shown that Flt-1 is a main receptor concerned in the migration of vascular endothelial cells.

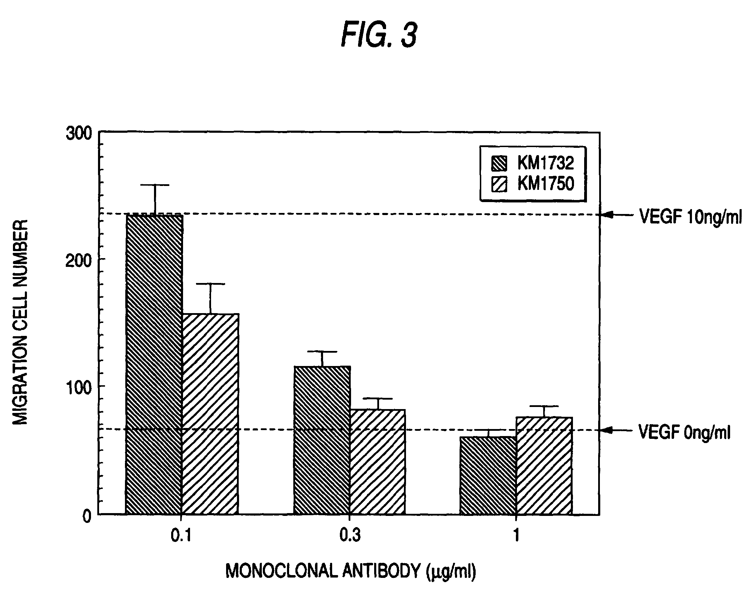

Fig. 3 shows a result of the comparison of activities of two anti-VEGF receptor Flt-1 monoclonal antibodies KM1750 and KM1732 to inhibit migration of vascular endothelial cells. These two monoclonal antibodies showed the activity to inhibit migration of vascular endothelial cells in a concentration dependent manner within a monoclonal antibody concentration of 0.1 to 1 µg/ml.

Based on the above, it was revealed that the migration of vascular endothelial cells induced by VEGF is completely inhibited by the anti-Flt-1 monoclonal antibodies.

Example 4

Northern blotting analysis of angiogenesis acceleration activation factors:

Examination was made on the effects of the two anti-VEGF receptors, KDR and Flt-1, monoclonal antibodies on the expression of mRNA corresponding to the molecules (ets-1, MMP-1, KDR and flt-1) which have been reported to have the activity to accelerate angiogenesis through their activation in vascular endothelial cells in the process of angiogenesis.

A medium of HUVEC cultured in a 6.0 cm dish until the cells became sub-confluent was changed with M-199 medium (3 ml) containing 5% FCS and the culturing was continued for 24 hours. After the culturing, the cells were pre-cultured for 15 minutes by adding each anti-VEGF receptor monoclonal antibody (final concentration 0, 1 or 10 µg/ml) and then cultured for 2 or 4 hours by adding VEGF (final concentration 1 nM). After the culturing, total RNA was extracted using ISOGEN (manufactured by Nippon Gene) in accordance with the protocol attached thereto. The Northern blotting analysis was carried out in accordance with the method of Iwasaki [*J. Cellular Physiology, 169*: 522 (1996)]. Human ets-1, MMP-1 and GAPDH labeled with $^{32}$P to be used as a probe, were prepared in accordance with the method of Iwasaka [*J. Cellular Physiology, 169*: 522 (1996)]. Human KDR and human flt-1 cDNA molecules were prepared by the reverse-transcriptional PCR reported by Iwasaka [*J. Cellular Physiology, 169*: 522 (1996)] using the synthetic primers of SEQ ID NOS:1 to 4 and the total RNA of HUVEC as the template. In this connection, SEQ ID NO:1 shows a sense primer of human KDR, SEQ ID NO:2 shows an antisense primer of human KDR, SEQ ID NO:3 shows a sense primer of human flt-1 and SEQ ID NO:4 shows an antisense primer of human flt-1.

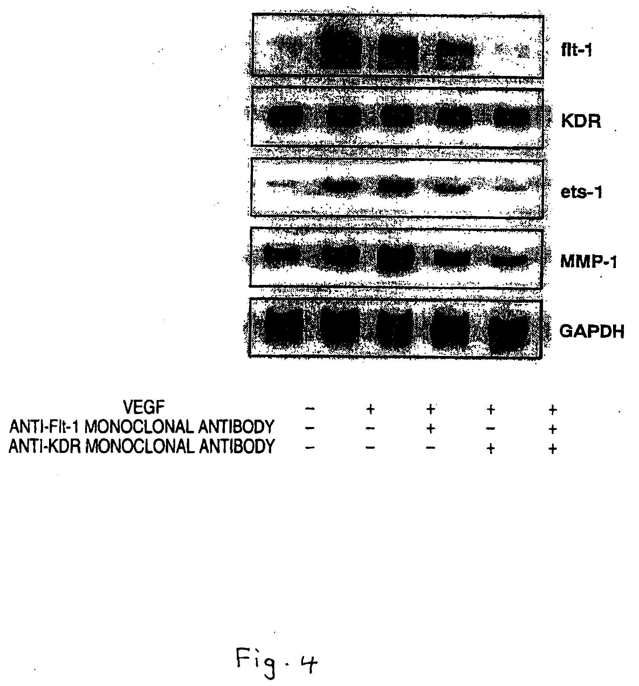

The results are shown in Fig. 4. The GAPDH as a standardized marker of the amount of total RNA in each lane showed equivalent signal in each lane. Expression of the flt-1 mRNA, ets-1 mRNA and MMP-1 mRNA was increased by the VEGF stimulation of HUVEC, but expression of KDR mRNA did not change. The flt-1 mRNA, ets-1 mRNA and MMP-1 mRNA whose expression was increased by the VEGF stimulation was not inhibited by anti-VEGF receptor Flt-1 monoclonal antibody KM1750 (final concentration 1 µg/ml) but partially inhibited by anti-VEGF receptor KDR monoclonal antibody KM1992 (final concentration 10 µg/ml). In addition, the flt-1 mRNA, ets-1 mRNA and MMP-1 mRNA whose expression was increased by the VEGF stimulation was completely inhibited to the expression level at the time of nonstimulation of VEGF, by using anti-VEGF receptor Flt-1 monoclonal antibody KM1750 (final concentration 1 µg/ml) and anti-VEGF receptor KDR monoclonal antibody KM1992 (final concentration 10 µg/ml) in combination.

Thus, it was shown that KDR is a main receptor concerned in the expression induction of flt-1 mRNA, ets-1 mRNA and MMP-1 mRNA by the VEGF stimulation of HUVEC, and Flt-1 has a role in accelerating the expression induction mediated by KDR.

Based on the above, it was revealed that expression induction of the flt-1 mRNA, ets-1 mRNA and MMP-1 mRNA by VEGF stimulation is inhibited by the anti-KDR monoclonal antibody, and a synergistic expression induction inhibition effect is obtained by use in combination with the anti-Flt-1 monoclonal antibody.

Example 5

Western blotting analysis of angiogenesis acceleration activation factors:

Examination was made on the effects of the two anti-VEGF receptors, KDR and Flt-1, monoclonal antibodies on the protein expression of the molecules (p38, ERK-1, ERK-2, JNK-1 and JNK-2) which have been reported to have the activity to accelerate angiogenesis through their activation in the process of angiogenesis. A medium of HUVEC cultured in a 6.0 cm dish until the cells became sub-confluent was changed with M-199 medium (3 ml) containing 5% FCS and the culturing was continued for 24 hours. After the culturing, the cells were pre-cultured for 15 minutes by adding each anti-VEGF receptor monoclonal antibody (final concentration 0, 1 or 10 µg/ml) and then cultured for 5 minutes by adding VEGF (final concentration 1 nM). After the culturing, RIPA buffer (1 mM sodium orthovanadate, 50 mM NaF, 5 mM b-glycerophosphate, 10 mM sodium pyrophosphate, 5 mM EDTA, 1 mM PMSF, 1 mg/ml leupeptin, 1 mg/ml pepstatin) was added to the cells, and the cells were peeled off from the dish and lysed at 4°C for 30 minutes. The cell lysate was centrifuged (15,000 rpm) at 4°C for 15 minutes, and the supernatant was recovered as a cell extract. The cell, extract was examined by Western blotting analysis in accordance with the method of Iwasaka [*J. Cellular Physiology, 169*: 522 (1996)]. Regarding the detection antibodies, rabbit anti-ACTIVE APK serum (manufactured by Promega), rabbit anti-ACTIVE JNK serum (manufactured by Promega) or rabbit anti-ACTIVE p38 serum (manufactured by Promega) was used as the primary antibody and horseradish peroxidase-labeled protein G (manufactured by Bio-Rad) was used as the secondary antibody, and a band reacted with each antibody was detected using ECL System (manufactured by Amersham).

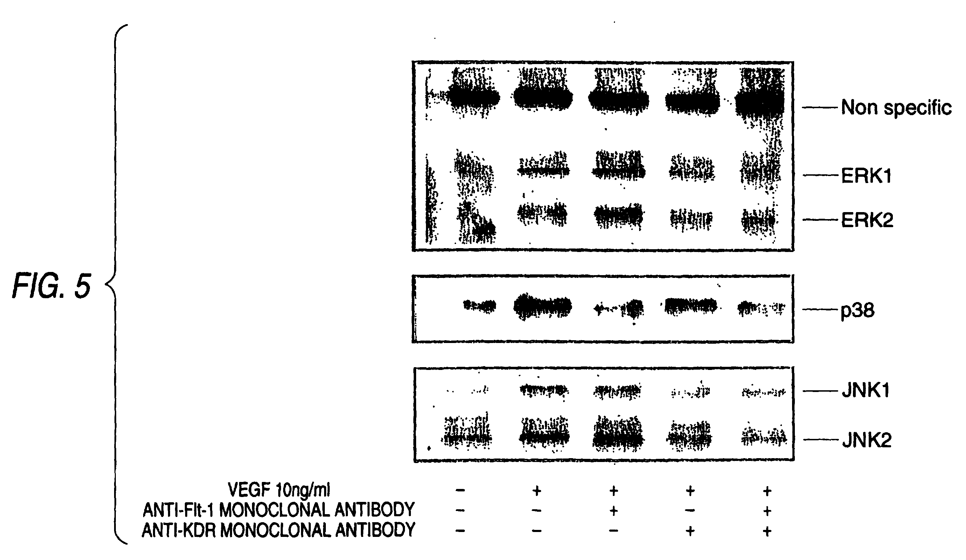

The results are shown in Fig. 5. As shown in Fig. 5, increase in the expression of p38 was observed by the VEGF stimulation of HUVEC. The increase in the expression of p38 was completely inhibited to the expression level at the time of nonstimulation of VEGF, by anti-VEGF receptor Flt-1 monoclonal antibody KM1750 (final concentration 1 μg/ml). On the other hand, a partial weak inhibition activity was observed by anti-VEGF receptor KDR monoclonal antibody KM1992 (final concentration 10 μg/ml). Increase in the inhibition activity was not found when anti-VEGF receptor Flt-1 monoclonal antibody KM1750 (final concentration 1 μg/ml) and anti-VEGF receptor KDR monoclonal antibody KM1992 (final concentration 10 μg/ml) are used in combination. Thus, it was shown that Flt-1 is a main receptor concerned in the expression induction of p38. That is, it was revealed that the expression induction of p38 by VEGF can be completely inhibited by the anti-Flt-1 monoclonal antibody.

On the other hand, as shown in Fig. 5, increase in the expression of ERK-1, ERK-2, JNK-1 and JNK-2 was found by the VEGF stimulation of HUVEC. The increase in the expression of ERK-1, ERK-2, JNK-1 and JNK-2 was completely inhibited to the expression level at the time of nonstimulation of VEGF, by anti-KDR monoclonal antibody KM1992 (final concentration 10 μg/ml). On the other hand, anti-VEGF receptor Flt-1 monoclonal antibody KM1750 (final concentration 1 μg/ml) showed completely no inhibition activity. Also, increase in the inhibition activity was not found when anti-VEGF receptor Flt-1 monoclonal antibody KM1750 (final concentration 1 μg/ml) and anti-VEGF receptor KDR monoclonal antibody KM1992 (final concentration 10 μg/ml) were used. Thus, it was shown that KDR is a main receptor concerned in the expression induction of ERK1, ERK2, JNK1 and JNK2.

Based on the above, it was revealed that expression induction of ERK1, ERK2, JNK1 and JNK2 by VEGF can be inhibited completely by the anti-KDR monoclonal antibody.

Example 6

Tests on cell-spreading, actin stress fiber formation and focal contact formation:

Examination was made on the effects of the two anti-VEGF receptors, KDR and Flt-1, monoclonal antibodies on the cell-spreading, actin stress fiber formation and focal contact formation which are observed in vascular endothelial cells activated in the process of angiogenesis.

Cells of HUVEC ($3 \times 10^4$) were suspended in the M-199 medium containing 5% FCS, inoculated into a dish (35 mm) coated with type I collagen and cultured at 37°C for 2 hours. Subsequently, the culturing was continued for 15 minutes by adding each anti-VEGF receptor monoclonal antibody (final concentration 0, 1 or 10 μg/ml). After the culturing, they were cultured for 15 minutes by adding VEGF (final concentration 10 ng/ml). After the culturing, the cells were fixed with 3.7% formaldehyde and then PBS containing 0.1% NP40 was added thereto to increase membrane permeability of the cells. Next, after blocking nonspecific binding sites with PBS containing 1% BSA, F-actin was detected in accordance with the method of Nehls *et al*. [*Microvascular Research, 42*: 103 (1991)] using rhodamine-conjugated phalloidin. Vinculin was detected in accordance with the method of Kellie *et al*. [*Experimental Cell Research, 160*: 259 (1985)] by an indirect fluorescent antibody technique using anti-vinculin monoclonal antibody (manufactured by Seikagaku Kogyo) and FITC-labeied anti-mouse antibody (manufactured by Jackson ImmunoResearch Laboratories). Confocal microscopy, LSM410 (manufactured by Carl Zeiss) was used in the detection of F-actin and vinculin. Size of the cells was analyzed using NIH image program.

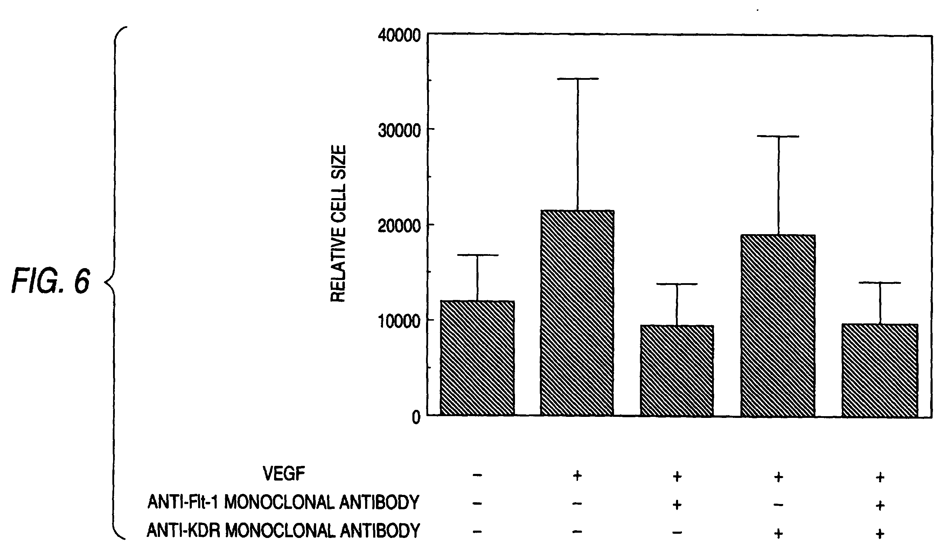

The results are shown in Table 1 and Fig. 6.

Table 1

| Samples added | VEGF | - | + | + | + |
|---|---|---|---|---|---|
| | Anti-Flt-1 monoclonal antibody | - | - | + | - |
| | Anti-KDR monoclonal antibody | - | - | - | + |
| Changes in cell morphology | Actin stress fiber formation | - | + | - | + |
| | Focal contact formation | - | + | + | - |

As shown in Table 1, although actin stress fiber formation and focal contact formation of HUVEC were accelerated by the VEGF stimulation, the actin stress fiber formation was selectively inhibited by anti-VEGF receptor Flt-1 monoclonal antibody KM1750 (final concentration 1 μg/ml), while the focal contact formation was selectively inhibited by anti-VEGF receptor KDR monoclonal antibody KM1992 (final concentration 10 μg/ml).

As shown in Fig. 6, the size of HUVEC became large by the VEGF stimulation and was selectively inhibited by anti-VEGF receptor Flt-1 monoclonal antibody KM1750 (final concentration 1 μg/ml) but not inhibited by anti-VEGF receptor KDR monoclonal antibody KM1992 (final concentration 10 μg/ml). Thus, it was shown that KDR is a main receptor concerned in the focal contact formation, while Flt-1 is a main receptor concerned in the actin stress fiber formation and cell enlargement.

Based on the above, it was revealed that the focal contact formation by VEGF can be inhibited by the anti-KDR monoclonal antibody and that the actin stress fiber formation and cell enlargement by VEGF can be inhibited by the anti-Flt-1 monoclonal antibody.

Reference Example 1

1. Preparation of antigen

(1) Construction of soluble human VEGF receptor Flt-1 3N expression vector

A vector was produced in the following manner, for use in the expression of a soluble human VEGF receptor Flt-1 fragment (hereinafter referred to as "soluble human VEGF receptor Flt-1 3N") which corresponds to a region of the 1st to 338th amino acids (including a signal sequence) from the N-terminal of human VEGF receptor Flt-1. The soluble human VEGF receptor Flt-1 3N corresponds to the three immunoglobulin-like regions from the N-terminal of the extracellular domain of the soluble human VEGF receptor Flt-1.

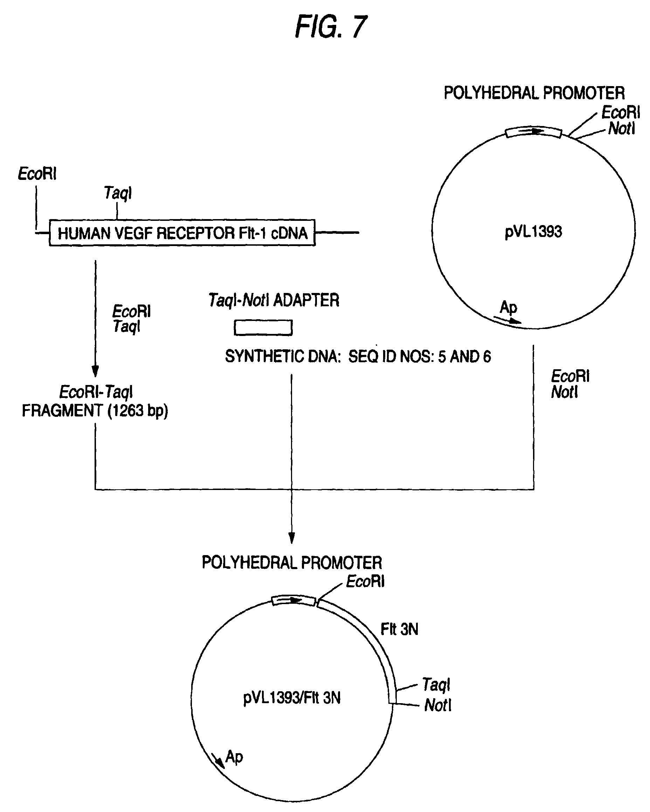

A cDNA clone flt#3-7 [M. Shibuya et al., Oncogene, 5: 519 (1990)] which contains full length cDNA encoding the human VEGF receptor Flt-1 was partially digested with restriction enzymes EcoRI and TaqI to collect a 1,263 bp EcoRI-TaqI DNA fragment from the 5'-end, and the thus collected fragment was inserted into the 5' side EcoRI site and 3' side NotI site downstream of the transcription initiation point of the polyhedrin gene of a baculovirus gene recombinant vector pVL1393 plasmid (manufactured by In Vitrogen) using a TaqI-NotI adapter into which a termination codon had been artificially introduced (a synthetic DNA fragment having the nucleotide sequences shown in the SEQ ID NO:5 and SEQ ID NO:6) to obtain soluble human VEGF receptor Flt-1 3N expression vector pVL1393/Flt 3N (Fig. 7).

(2) Construction of soluble human VEGF receptor Flt-1 7N expression vector

A vector was produced in the following manner, for use in the expression of a soluble human VEGF receptor Flt-1 fragment (referred to as "soluble human VEGF receptor Flt-1 7N" hereinafter) which corresponds to a region of the 1st to 750th amino acids (including a signal sequence) from the N-terminal of human VEGF receptor Flt-1. The soluble human VEGF receptor Flt-1 7N corresponds to the seven immunoglobuiin-like regions of the extracellular domain of the soluble human VEGF receptor Flt-1.

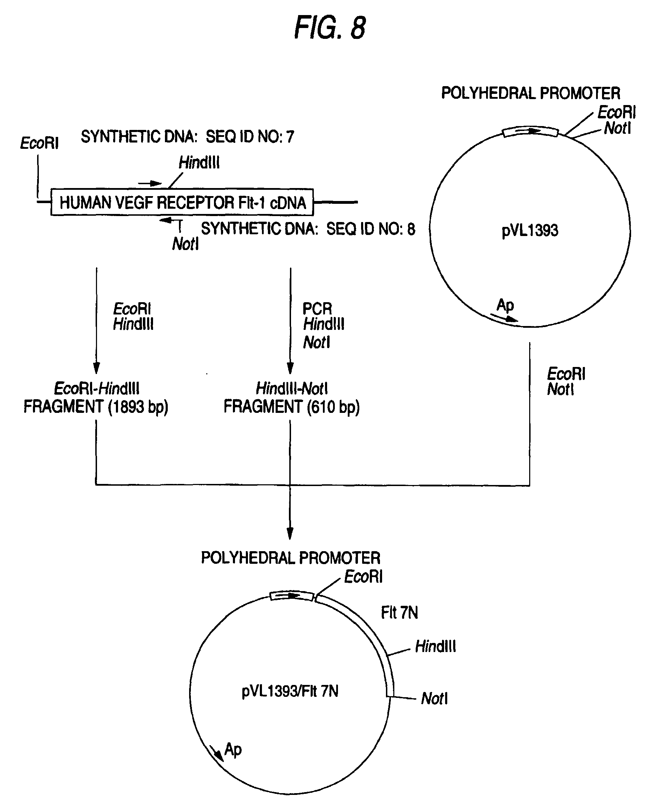

A 2.5 unit portion of Taq polymerase was added to 100 μl of 0.001% (w/v) gelatin solution of 10 mM MgCl$_2$ containing 10 pmol of primers having the nucleotide sequences shown in SEQ ID NO:7 and SEQ ID NO:8, 10 ng of flt#3-7 clone [Oncogene, 5: 519 (1990)] DNA and 10 mM deoxynucleotide triphosphates. The polymerase chain reaction (PCR) was repeated 30 times in which one reaction consisted, after pretreatment at 95°C for 5 minutes, of treatments at 95°C for 90 seconds, at 50°C for 90 seconds and finally at 72°C for 90 seconds, subsequently collecting a DNA fragment. The DNA fragment was digested with HindIII (the 1893 bp position in the flt#3-7 clone) and NotI to obtain a 610 bp HindIII-NotI DNA fragment, namely a DNA fragment containing a 1894-2499 bp fragment of the flt#3-7 clone, termination codon and NotI recognition sequence. Next, the flt#3-7 clone was digested with restriction enzymes EcoRI and HindIII to collect an EcoRI-HindIII fragment of 1893 bp from the 5'-end. The 610 bp HindIII-NotI DNA fragment and the 1893 bp EcoRI-HindIII fragments were then inserted into the 5' side EcoRI site and 3' side NotI site downstream of the transcription initiation point of the polyhedrin gene of a baculovirus gene recombinant, vector pVL1393 plasmid to produce soluble human VEGF receptor Flt-1 7N expression vector pVL1393/Flt 7N (Fig. 8).

(3) Production of recombinant virus for use in the expression of soluble human VEGF receptor Flt-1 in insect cells

For the production of protein by insect cells, it is necessary to produce a recombinant virus into which a gene of interest is integrated, and the production process consists of a step in which a cDNA molecule encoding a protein of interest is inserted into a special plasmid, which is called a transfer vector, and a subsequent step in which a wild type virus and the transfer vector are co-transfected into insect cells to obtain a recombinant virus by homologous recombination. These steps were carried out in the following manner using BaculoGold Starter Kit manufactured by Pharmigen (Product No. PM-21001K) in accordance with the attached manual.

A recombinant baculovirus was produced in the following manner by introducing a filamentous baculovirus DNA (BaculoGold baculovirus DNA, manufactured by Pharmigen) and the thus produced transfer vector DNA into insect cells Sf9 (manufactured by Pharmigen) which had been cultured using TMN-FH insect medium (manufactured by Pharmigen), using a lipofectin method [*Protein, Nucleic Acid, Enzyme*, 37: 2701 (1992)].

A 1 μg portion of pVL1393/Flt7N produced in (2) or pVL1393/Flt3N produced in (1) and 20 ng of filamentous baculovirus DNA were dissolved in 12 μl of distilled water, the solution was mixed with a mixture of 6 μl lipofectin and 6 μl distilled water and then the resulting mixture was allowed to stand at room temperature for 15 minutes. Separately from this, $1\times10^6$ of Sf9 cells were suspended in 2 ml of Sf900-II medium (manufactured by Gibco) and put into a cell culture plastic Petri dish of 35 mm in diameter. To this was added whole volume of the just described solution of plasmid DNA, filamentous baculovirus DNA and lipofectin mixture, followed by 3 days of culturing at 27°C to collect 1 ml of the culture supernatant containing the recombinant virus. A 1 ml portion of fresh Sf900-II medium was added to the resulting Petri dish and 3 days of culturing was carried out at 27°C to obtain an additional 1.5 ml of the culture supernatant containing the recombinant virus.

Next, the thus obtained recombinant virus for use in the protein expression was grown in the following manner.

A $2\times10^7$ portion of Sf9 cells were suspended in 10 ml of Sf900-II medium, put into a 175 $cm^2$ flask (manufactured by Greiner) and allowed to stand at room temperature for 1 hour to adhere the cells to the flask. The supernatant fluid was subsequently discarded and 15 ml of fresh TMN-FH insect medium and a 1 ml portion of the culture supernatant containing the recombinant virus described above were added and cultured for 3 days at 27°C. After the culturing, the supernatant fluid was centrifuged at 1,500 × g for 10 minutes to remove the cells to obtain a recombinant virus solution for use in the protein expression.

The titer of virus in the thus obtained recombinant virus solution was calculated by the method described in BaculoGold Starter Kit Manual (manufactured by Pharmigen).

A $6\times10^6$ portion of Sf9 cells were suspended in 4 ml of Sf900-II medium, put into a cell culture plastic Petri dishes of 60 mm in diameter and allowed to stand at room temperature for 1 hour to adhere the cells to the dish. Next, the supernatant fluid was discarded, 400 μl of fresh Sf900-II medium and the above-described recombinant virus solution diluted 10,000 times with Sf900-II medium were added to the dish and allowed to stand at room temperature for 1 hour, the medium was removed and then 5 ml of a medium containing 1% low melting point agarose (Agarplaque Agarose, manufactured by Pharmigen) (produced by mixing 1 ml of sterilized 5% Agarplaque plus agarose aqueous solution with 4 ml of TMN-FH insect medium and stored at 42°C) was poured into the dish. After standing at room temperature for 15 minutes, the dish was tied with a vinyl tape to prevent drying, put into a sealable plastic container and then cultured at 27°C for 6 days. A 1 ml portion of PBS containing 0.01% of Neutral Red was added to the dish to carry out the additional culturing for 1 day and then the number of the thus formed plaques was counted. By the above procedure, it was found that each of the recombinant virus solutions contained virus particles of about $1\times10^7$ PFU per ml.

(4) Expression of soluble human VEGF receptors Flt-1 7N and Flt-1 3N in insect cells and purification thereof

Soluble human VEGF receptors Flt-1 7N and Flt-1 3N were obtained in the following manner. A $4\times10^7$ portion of High Five cells were suspended in 30 ml of EX-CELL™ 400 medium (manufactured by JRH Biosciences) contained in a 175 $cm^2$ flask (manufactured by Greiner) and allowed to stand at room temperature for 1 hour to adhere the cells to the flask. A 1 ml portion of a solution containing about $1\times10^8$ to $3\times10^8$ PFU/ml of recombinant virus particles obtained in (3) derived from the transfer vectors pVL1393/Flt 7N and pVL1393/Flt 3N was added to the flask to carry out infection at room temperature for 2 hours. The culture supernatant was removed and 30 ml of fresh EX-CELL™ 400 medium was added to carry out 3 to 4 days of culturing at 27°C. After completion of the culturing, the culture supernatant was collected and centrifuged at 1,500 × g for 10 minutes to obtain a supernatant fluid.

A column was packed with about 60 ml of heparin-Sepharose CL-6B gel (manufactured by Pharmacia Biotech AB) and washed with 600 ml of 20 mM Tris-HCl (pH 7.5) buffer at a flow, rate of 0.5 ml/minute. After the washing, 1,000 ml of the culture medium containing soluble human VEGF receptors Flt-1 7N and Flt-1 3N, which had been prepared in the above-described manner, was passed through the heparin-Sepharose CL-6B column at a flow rate of 0.5 ml/minute. After washing with 600 ml of 20 mM Tris-HCl (pH 7.5) buffer at a flow rate of 0.5 ml/minute, 600 ml of 20 mM Tris-HCl (pH 7.5) buffer having a density gradient of 0 M to 1.1 M NaCl was passed through the column to carry out elution of the proteins adsorbed to the heparin-Sepharose, and the eluate was fractionated in 8 ml portions. Proteins contained in each fraction were analyzed by SDS polyacrylamide gel electrophoresis (SDS-PAGE), and 60 to 80 ml of fractions containing soluble human VEGF receptors Flt-1 7N and Flt-1 3N were collected and concentrated using CentriPrep 10 (manufactured by Amicon). After the concentration, soluble human Flt-1 7N and Flt-1 3N were obtained as solutions of 5 ml and 13 ml, respectively (protein concentrations were 331 μg/ml and 204 μg/ml).

(5) Confirmation of the purity of soluble human VEGF receptors Flt-1 7N and Flt-1 3N

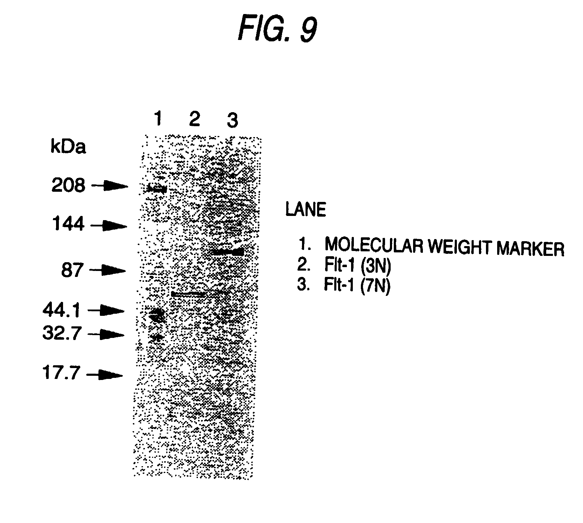

Purity of the thus purified soluble human VEGF receptors Flt-1 7N and Flt-1 3N was confirmed by SDS-PAGE. The SDS-PAGE was carried out in accordance with a known method [*Anticancer Research, 12*: 1121 (1992)]. Using a 5 to 20% gradient gel (manufactured by Atto) as the gel, electrophoresis of Flt-1 7N and Flt-1 3N, each 2 μg as protein per lane, was carried out under reducing conditions, and the resulting gel was stained with Coomassie Brilliant Blue. The results are shown in Fig. 9. Purity of Flt-1 7N and Flt-1 3N was found to be 95% or more.

(6) Purification of control antigen protein of soluble human VEGF receptors Flt-1 7N and Flt-1 3N

The control antigen protein (negative control protein) of soluble human VEGF receptors Flt-1 7N and Flt-1 3N was obtained in the following manner. A $4\times10^7$ portion of High Five cells were suspended in 30 ml of EX-CELL™ 400 medium (manufactured by JRH Biosciences) contained in a 175 cm$^2$ flask (manufactured by Greiner), allowed to stand at room temperature for 1 hour to adhere the cells to the flask and then cultured at 27°C for 3 to 4 days. After completion of the culturing, the culture supernatant was collected and centrifuged at $1,500 \times$ g for 10 minutes to obtain a supernatant fluid.
A column was packed with about 20 ml of heparin-Sepharose CL-6B gel (manufactured by Pharmacia Biotech AB) and washed with 200 ml of 20 mM Tris-HCl (pH 7.5) buffer at a flow rate of 0.5 ml/minute. After the washing, 500 ml of the culture medium of High Five cells was passed through the heparin-Sepharose CL-6B column at a flow rate of 0.5 ml/minute. After washing with 200 ml of 20 mM Tris-HCl (pH 7.5) buffer at a flow rate of 0.5 ml/minute, 200 ml of 20 mM Tris-HCl (pH 7.5) buffer containing 1 M NaCl was passed through the column to carry out elution of the protein adsorbed to the heparin-Sepharose. The 1 M NaCl elution fraction was concentrated using CentriPrep 10 (manufactured by Amicon) to obtain 7 ml of the control antigen protein (867 μg/ml as protein concentration).

(7) Confirmation of human VEGF binding activity of soluble human VEGF receptors Flt-1 7N and Flt-1 3N

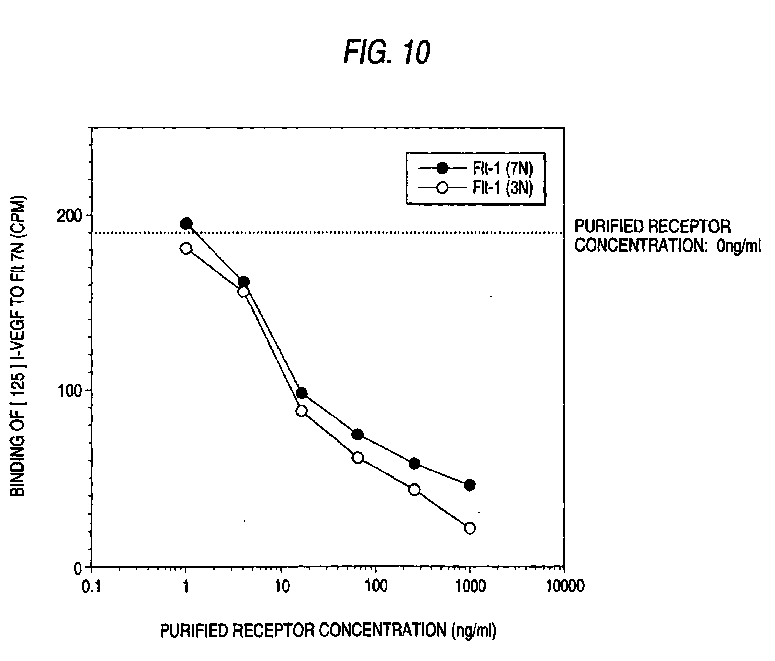

The human VEGF binding activity of soluble human VEGF receptors Flt-1 7N and Flt-1 3N was confirmed in the following manner.
Methanol was dispensed in 100 μl portions into wells of a 96 well Immobilon™-P Filtration Plate (manufactured by Millipore) to give a hydrophilic nature to the PVDF membrane on the bottom of the plate. After washing with water, the soluble human Flt-1 7N diluted with PBS to a concentration of 2 μg/ml was dispensed in 50 μl/well portions and allowed to stand overnight at 4°C for its absorption. After washing, PBS containing 1% bovine serum albumin (BSA) was dispensed in 100 μl/well portions and the reaction was carried out at room temperature for 1 hour to block the remaining active residues. After washing with PBS, each of the purified soluble human VEGF receptors Flt-1 7N and Flt-1 3N obtained in (4) was dispensed in 50 μl/well portions (final concentration, 1 to 1,000 ng/ml) and then $^{125}$I-labeled human VEGF (final concentration, 3 ng/ml: manufactured by Amersham) was dispensed in 50 μl/well portions, subsequently carrying out the reaction at room temperature for 1.5 hours. After washing with 0.05% Tween-PBS, the wells were dried at 50°C, and Microscinti-0 (manufactured by Packard) was dispensed in 20 μl/well portions to measure the radioactivity of the $^{125}$I-labeled human VEGF bound to each well using Top Count (manufactured by Packard).
The results are shown in Fig. 10. It was shown that soluble human VEGF receptors Flt-1 7N and Flt-1 3N inhibit binding of $^{125}$I-labeled human VEGF to soluble human VEGF receptor Flt-1 7N in a concentration dependent manner. Since the soluble human VEGF receptors Flt-1 7N and Flt-1 3N showed similar degree of the human VEGF binding activity, it was revealed that the human VEGF binds to the Flt-1 3N moiety (the 1st to 338th amino acids from the N-terminal including signal sequence).

(8) Expression of human VEGF in insect cells

The human VEGF was obtained in the following manner. A $4\times10^7$ portion of High Five cells were suspended in 30 ml of EX-CELL™ 400 medium (manufactured by JRH Biosciences) contained in a 175 cm$^2$ flask (manufactured by Greiner) and allowed to stand at room temperature for 1 hour to adhere the coils to the flask. A 1 ml portion of a solution containing about $1\times10^8$ to $3\times10^8$ PFU/ml of human VEGF recombinant baculovirus particles obtained in accordance with the known method [*Cell Growth & Differentiation, 7*: 213 (1996)] was added to the flask to carry out infection at room temperature for 2 hours. The culture supernatant was removed and 30 ml of fresh EX-CELL™ 400 medium was added to carry out 3 to 4 days of culturing at 27°C. After completion of the culturing, the culture supernatant was collected and centrifuged at $1,500 \times$ g for 10 minutes to obtain a supernatant fluid.
A column was packed with about 40 ml of heparin-Sepharose CL-6B gel (manufactured by Pharmacia Biotech AB) and washed with 400 ml of 20 mM Tris-HCl (pH 7.5) buffer at a flow rate of 0.5 ml/minute. After washing, 1,500 ml of the culture medium containing the human VEGF prepared in the above-described manner was passed through

EP 1 086 705 A1

the heparin-Sepharose CL-6B column at a flow rate of 0.5 ml/minute. After washing with 400 ml of 20 mM Tris-HCl (pH 7.5) buffer at a flow rate of 0.5 ml/minute, 120 ml of each of 20 mM Tris-HCl (pH 7.5) buffers containing 0.2 M, 0.5 M and 1 M NaCl was passed through the column in this order to carry out stepwise elution of the proteins adsorbed to the heparin-Sepharose, and the eluate was fractionated in 8 ml portions. Proteins contained in each fraction were analyzed by SDS polyacrylamide gel electrophoresis, and 120 ml of fractions (0.5 to 1 M NaCl fractions) containing human VEGF were collected. After concentration using CentriPrep-10 (manufactured by Amicon), human VEGF was obtained as 4 ml of solution (protein concentration, 1.2 mg/ml).

2. Immunization of animals and preparation of antibody-producing cells

A 50 $\mu$g portion of each of the antigens obtained in 1(4) was administered, together with 2 mg of aluminum hydroxide gel and $1\times10^9$ cells of pertussis vaccine (manufactured by Chiba Serum Institute), into 5-week-old female BALB/c mice (SLC Japan), B6C3F1 mice (Charles River Japan) or female SD rats (SLC Japan), and, starting on 2 weeks thereafter, 10 to 50 $\mu$g of the protein was administered once a week for a total of four times. Also, $1\times10^7$ of NIH3T3-Flt-1 cells were administered 6 times into three, 5 week old female BALB/c (SLC Japan) mice. Blood samples were collected from the fundus of the eye or the caudal vein, their serum antibody titers were examined by the enzyme immunoassay described in the following, and spleens were excised from mice or rats showing sufficient antibody titer 3 days after the final immunization. In this connection, immunization was not induced in the 5-week-old female BALB/c to which NIH3T3-Flt-1 cells were administered, so that the antibody titer upon soluble Flt-1 7N was not increased.

The thus excised spleen was cut to pieces in MEM medium (manufactured by Nissui Pharmaceutical), unbound using a pair of forceps and then centrifuged (1,200 rpm for 5 minutes). The resulting supernatant was discarded, and the thus obtained sediment was treated with Tris-ammonium chloride buffer (pH 7.65) for 1 to 2 minutes to eliminate erythrocytes, washed three times with MEM medium and used in cell fusion.

3. Enzyme immunoassay

With regard to the measurement of antisera derived from mice or rats immunized with the soluble human Flt-1 7N and Flt-1 3N obtained in 1(4) and culture supernatants of hybridomas, the soluble human VEGF receptors Flt-1 7N and Flt-1 3N obtained from the insect cell culture supernatant of 1(4) were used as antigens. A 1 to 10 $\mu$g/ml PBS-diluted solution of each of the soluble human VEGF receptors Flt-1 7N and Flt-1 3N and the heparin column absorption fraction of High Five cell culture supernatant obtained in 1(6) as a control antigen was dispensed in 50 $\mu$l/well portions into a 96 well plate for EIA (manufactured by Greiner) and allowed to stand overnight at 4°C for coating. After washing, PBS containing 1% bovine serum albumin (BSA) was dispensed in 100 $\mu$l/well portions and the reaction was carried out at room temperature for 1 hour to block the remained active residues. After discarding 1% BSA-PBS, antiserum of immunized mouse or immunized rat and culture supernatant of a hybridoma were dispensed in 50 $\mu$l/well portions to carry out the reaction for 2 hours. After washing with 0.05% Tween-PBS, peroxidase-labeled rabbit anti-mouse immunoglobulin or peroxidase-labeled rabbit anti-rat immunoglobulin (both manufactured by DAKO) was dispensed in 50 $\mu$l/well portions and the reaction was carried out at room temperature for 1 hour, the plate was washed with 0.05% Tween-PBS and then color development was caused using ABTS substrate solution [2,2-azinobis(3-ethylbenzothiazole-6-sulfonic acid) ammonium salt] to measure maximum absorbance at OD415nm using E max (manufactured by Molecular Devices).

4. Preparation of mouse myeloma cells

8-Azaguanine-resistant mouse myeloma cell line P3U1 was cultured using normal medium to secure $2\times10^7$ or more of the cells for use in cell fusion as the parent cell line.

5. Production of hybridoma

The mouse spleen cells or rat spleen cells obtained in 2 and the myeloma cells obtained in 4 were mixed to a ratio of 10:1 and centrifuged (1,200 rpm for 5 minutes), the supernatant was discarded, the precipitated cells were thoroughly loosened to which, while stirring at 37°C, were subsequently added a mixed solution of 2 g polyethylene glycol-1000 (PEG-1000), 2 ml MEM medium and 0.7 ml DMSO in an amount of 0.2 to 1 ml/$10^8$ mouse myeloma cells and then 1 to 2 ml of MEM medium several times at 1 to 2 minute intervals, and then the total volume was adjusted to 50 ml by adding MEM medium. After centrifugation (900 rpm for 5 minutes), the supernatant was discarded and the thus obtained cells were gently loosened and then gently suspended in 100 ml of HAT medium by repeated drawing up into and discharging from a graduated pipette.

The suspension was dispensed in 100 $\mu$l portions into wells of a 96 well culture plate and cultured at 37°C for 10 to 14 days in an atmosphere of 5% $CO_2$ in a 5% $CO_2$ incubator. The resulting culture supernatant was examined by the enzyme immunoassay described in 2(3) of Example 1 to select wells which reacted specifically with the soluble human VEGF receptor Flt-1 7N or Flt-1 3N obtained in 1(4) but did not react with the control antigen obtained in 1(6), and then cloning was repeated twice by changing the medium to HT medium and normal medium to establish hybridomas capable of producing anti-human VEGF receptor Flt-1 monoclonal antibodies. The results are shown in Table 2 below.

Table 2

| Animal | Head | Immunogen | Screening source | Wells screened | The number of hybridomas established |
|---|---|---|---|---|---|
| Balb/c mouse | 3 | NIH3T3-Flt-1 | Flt 7N | - | - |
| SD rat | 1 | Flt 7N | Flt 7N | 1008 | 3 (KM1733,1735,1736) |
| Balb/c mouse | 1 | Flt 7N | Flt 7N | 672 | 5 (KM1737,1739,1740, 1742,1743) |
| SD rat | 1 | Flt 7N | Flt 7N | 1176 | 3 (KM1745,1746,1747) |
| B3C3F1 mouse | 1 | Flt 7N | Flt 3N | 672 | 3 (KM1748,1749,1750) |
| Balb/c mouse | 1 | Flt 7N | Flt 3N | 420 | 3 (KM1730,1731,1732) |

When hybridomas obtained from one Balb/c mouse and two SD rats immunized with the soluble human VEGF receptor Flt-1 7N obtained in 1(4) were screened for about 672 wells and about 2,184 wells, respectively, using the soluble human VEGF receptor Flt-1 7N, respective 5 clones and 6 clones of anti-human VEGF receptor Flt-1 monoclonal antibodies were obtained, and they were named KM1737, KM1739, KM1740, KM1742 and KM1743 and KM1733, KM1735, KM1736, KM1745, KM1746 and KM1747, respectively. None of these clones showed the activity to inhibit binding of human VEGF to Flt-1 as shown 8. Additionally, KM1735, KM1736, KM1742, KM1743 and KM1745 reacted with human VEGF receptor Flt-1 expression cells by the immunocyte staining method described in the following 10, but the reaction was extremely weak in comparison with KM1730, KM1731 and KM1732.

On the other hand, when hybridomas obtained from one B3C3F1 mouse and one Balb/c mouse immunized with the soluble human VEGF receptor Flt-1 7N obtained in 1(4) were screened for about 672 wells and about 420 wells, respectively, using the soluble human VEGF receptor Flt-1 3N, 3 clones for each of anti-human VEGF receptor Flt-1 monoclonal antibodies were obtained, and they were named KM1748, KM1749 and KM1750 and KM1730, KM1731 and KM1732, respectively. Of these clones, three clones KM1732, KM1748 and KM1750 showed the activity to inhibit binding of human VEGF to Flt-1 as shown in the following 8. Additionally, three clones KM1730, KM1731 and KM1732 reacted markedly strongly with human VEGF receptor Flt-1 expression cells by the immunocyte staining method described in the following 10.

The antibody class of these monoclonal antibodies was determined by enzyme immunoassay using Subclass Typing Kit (manufactured by Zymed). The results are shown in the following Table 3.

Table 3

| Monoclonal antibody | Antibody subclass |
|---|---|
| KM1733 | mouse IgG2a |
| KM1735 | rat IgG1 |
| KM1736 | rat IgG2a |
| KM1737 | mouse IgG1 |
| KM1739 | mouse IgG1 |
| KM1740 | mouse IgG1 |
| KM1742 | mouse IgG1 |
| KM1743 | mouse IgG1 |

Table 3 (continued)

| Monoclonal antibody | Antibody subclass |
| --- | --- |
| KM1745 | rat IgG2a |
| KM1746 | rat IgG1 |
| KM1747 | rat IgG1 |
| KM1748 | mouse IgG2b |
| KM1749 | mouse IgG1 |
| KM1750 | mouse IgG2b |
| KM1730 | mouse IgG1 |
| KM1731 | mouse IgG2a |
| KM1732 | mouse IgG1 |

All of the monoclonal antibodies established in the present invention were IgG class.

Reference Example 2

Production of anti-human VEGF receptor KDR monoclonal antibody

1. Preparation of antigen

Various derivatives of soluble human VEGF receptor KDR-Fc and various soluble human VEGF receptor KDR were prepared as antigens in the following manner.

(1) Construction of expression vector for fusion gene of soluble human VEGF receptor KDR-7N with human antibody Fc region

A vector was prepared in the following manner for use in the expression of a fusion protein composed of a soluble human VEGF receptor KDR fragment which corresponds to the 19 amino acids described in SEQ ID NO:35 constituting the signal peptide of human VEGF receptor KDR and the amino acid sequence of the 1st to 738th positions described in SEQ ID NO:34 as mature human VEGF receptor KDR, a linker consisting of 6 amino acid residues (linker #1) and 227 amino acids that constitute a human antibody Fc region (hereinafter referred to as "soluble human VEGF receptor KDR-7N-Fc"). The soluble human VEGF receptor KDR-7N-Fc corresponds to a fusion protein which is composed of seven immunoglobulin-like domains from the N-terminal side of the extracellular region of soluble human VEGF receptor KDR, a linker consisting of 6 amino acid residues (linker #1) and a human antibody Fc region.

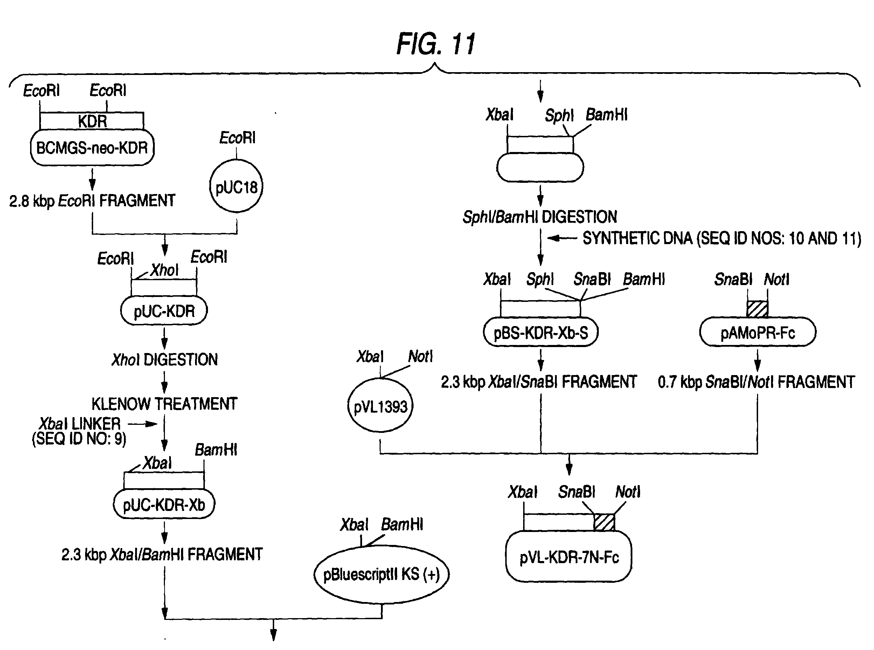

A cDNA clone BCMGS-neo-KDR [*Cell Growth & Differentiation, 7*: 213-221 (1996)] encoding the full length cDNA of human VEGF receptor KDR was digested with *Eco*RI, and a fragment of about 2.8 kb coding for the extracellular region and membrane-binding region of KDR was inserted into the *Eco*RI site of pUC18 to prepare pUC-KDR. The pUC-KDR was digested with *Xho*I and subjected to Klenow treatment and then an *Xba*I linker (SEQ ID NO:9) was inserted to prepare pUC-KDR-Xb. An *Xba*I-*Bam*HI (2.3 kbp) fragment of the pUC-KDR-Xb was inserted, into the *Xba*I/*Bam*HI site of pBluescriptII KS(+), and then a *Sph*I-*Bam*HI (5.2 kbp) fragment was prepared and synthetic linkers containing *Sna*BI site (SEQ ID NO:10 and SEQ ID NO:11) were inserted to prepare pBS-KDR-Xb-S. An *Xba*I/*Sna*BI (2.3 kbp) fragment of the pBS-KDR-Xb-S and a human antibody Fc region-encoding *Sna*BI/*Not*I (0.7 kbp) fragment on a plasmid pAMoPRFc [*The Journal of Immunology, 158*: 707-714 (1997)] were integrated into the downstream 5'-side *Xba*I and 3'-side *Not*I sites of the transcription initiation point of the polyhedrin gene of a baculovirus recombinant pVL1393 plasmid to construct an expression vector pVL-KDR-7N-Fc for a fusion gene of soluble human VEGF receptor KDR-7N with human antibody Fc region (Fig. 11).

(2) Construction of expression vector for fusion gene of soluble human VEGF receptor KDR-6N with human antibody Fc region

A vector was prepared in the following manner for use in the expression of a fusion protein composed of a soluble human VEGF receptor KDR fragment which corresponds to the 19 amino acids described in SEQ ID NO:35 constituting the signal peptide of human VEGF receptor KDR, the amino acid sequence of the 1st to 638th positions described in SEQ ID NO:34 as mature human VEGF receptor KDR, a linker consisting of 6 amino acid residues (linker #1) and 227 amino acids that constitute a human antibody Fc region (hereinafter referred to as "soluble human VEGF receptor KDR-6N-Fc"). The soluble human VEGF receptor KDR-6N-Fc corresponds to a fusion protein which is composed of six immunoglobulin-like domains from the N-terminal side of the extracellular region of soluble human VEGF receptor KDR, a linker consisting of 6 amino acid residues (linker #1) and a human antibody Fc region.

A 2.5 unit portion of Taq polymerase was added to 100 µl of 10 mM $MgCl_2$ 0.001% (W/V) gelatin solution containing 10 pmol of primers having the nucleotide sequences shown in SEQ ID NO:12 and SEQ ID NO:13, 10 ng of the pBS-KDR-Xb-S (see Preparation of antigen (1)) DNA and 10 mM of deoxynucleotide triphosphates. The reaction was carried out as pretreatment at 95°C for 5 minutes and then 30 cycles of polymerase chain reaction (PCR) were repeated, each cycle consisting of 95°C for 90 seconds, 50°C for 90 seconds and finally 72°C for 90 seconds to recover a DNA fragment. This DNA fragment was digested with EcoT221 and SnaBI to obtain a 80 bp EcoT221/SnaBI fragment. This DNA fragment and an EcoT221/SnaBI (5.2 kbp) fragment of the pBS-KDR-Xb-S (see Preparation of antigen (1)) were ligated to prepare pBS-KDR(6N)L. An XbaI/SnaBI (2.0 kbp) fragment of the pBS-KDR(6N)L and a human antibody Fc region-encoding SnaBI/NotI (0.7 kbp) fragment on pAMoPRFc (see Preparation of antigen (1)) were integrated into the downstream 5'-side XbaI and 3'-side NotI sites of the transcription initiation point of the polyhedrin gene of a baculovirus recombinant pVL1393 plasmid to construct an expression vector pVL-KDR-6N-Fc for a fusion gene of soluble human VEGF receptor KDR-6N with human antibody Fc region.

(3) Construction of expression vector for fusion gene of soluble human VEGF receptor KDR-5N with human antibody Fc region

A vector was prepared in the following manner for use in the expression of a fusion protein composed of a soluble human VEGF receptor KDR fragment which corresponds to the 19 amino acids described in SEQ ID NO:35 constituting the signal peptide of human VEGF receptor KDR, the amino acid sequence of the 1st to 518th positions described in SEQ ID NO:34 as mature human VEGF receptor KDR, a linker consisting of 6 amino acid residues (linker #1) and 227 amino acids that constitute a human antibody Fc region (hereinafter referred to as "soluble human VEGF receptor KDR-5N-Fc"). The soluble human VEGF receptor KDR-5N-Fc corresponds to a fusion protein which is composed of five immunoglobulin-like domains from the N-terminal side of the extracellular region of soluble human VEGF receptor KDR, a linker consisting of 6 amino acid residues (linker #1) and a human antibody Fc region.

An EcoRI/HincII (1.9 kbp) fragment of pUC-KDR-Xb and synthetic linkers having the nucleotide sequences of SEQ ID NO:14 and SEQ ID NO:15 were inserted into to the EcoRI/NotI site of a vector pBluescriptII SK(-) to construct pBS-KDR-5N. An XbaI-SnaBI (1.9 kbp) fragment of the pBS-KDR-5N and a human antibody Fc region-encoding SnaBI-NotI (0.7 kbp) fragment on a plasmid pAMoPRFc (see Preparation of antigen (1)) were integrated into the downstream 5'-side XbaI and 3'-side NotI sites of the transcription initiation point of the polyhedrin gene of a baculovirus recombinant pVL1393 plasmid to construct an expression vector pVL-KDR-5N-Fc for a fusion gene of soluble human VEGF receptor KDR-5N with human antibody Fc region.

(4) Construction of expression vector for fusion gene of soluble human VEGF receptor KDR-4N with human antibody Fc region

A vector was prepared in the following manner for use in the expression of a fusion protein composed of a soluble human VEGF receptor KDR fragment which corresponds to the 19 amino acids described in SEQ ID NO:35 constituting the signal peptide of human VEGF receptor KDR, the amino acid sequence of the 1st to 393rd positions described in SEQ ID NO:34 as mature human VEGF receptor KDR, a linker consisting of 2 amino acid residues (linker #2) and 227 amino acids that constitute a human antibody Fc region (hereinafter referred to as "soluble human VEGF receptor KDR-4N-Fc"). The soluble human VEGF receptor KDR-4N-Fc corresponds to a fusion protein which is composed of four immunoglobulin-like domains from the N-terminal side of the extracellular region of soluble human VEGF receptor KDR, a linker consisting of 2 amino acid residues (linker #2) and a human antibody Fc region.

A 2.5 unit portion of Taq polymerase was added to 100 µl of 10 mM $MgCl_2$ 0.001% (W/V) gelatin solution containing 10 pmol of primers having the nucleotide sequences shown in SEQ ID NO:16 and SEQ ID NO:17, 10 ng of the pUC-KDR-Xb DNA and 10 mM of deoxynucleotide triphosphates. The reaction was carried out as pretreatment at 95°C for 5 minutes and then 30 cycles of polymerase chain reaction (PCR) were repeated, each cycle consisting of 95°C for 90 seconds, 50°C for 90 seconds and finally 72°C for 90 seconds to recover a DNA fragment. This DNA fragment was digested with HindIII and KpnI to obtain a 520 bp HindIII-KpnI fragment. The DNA fragment and a human antibody Fc region-encoding KpnI/NotI (0.7 kbp) fragment on pAMoPRFc were inserted into the HindIII/NotI site of the vector pAMoPRFc (see Preparation of antigen (1)) to construct pAMo-4N-Fc. A HindIII/NotI (1 kbp) fragment of the pAMo-4N-Fc and an XbaI/HindIII (0.7 kbp) fragment of pUC-KDR-Xb were integrated into the downstream 5'-side XbaI and 3'-side NotI sites of the transcription initiation point of the polyhedrin gene of the baculovirus recombinant pVL1393 plasmid to construct an expression vector pVL-KDR-4N-Fc for a fusion gene of soluble human VEGF receptor KDR-4N with human antibody Fc region.

(5) Construction of expression vector for a fusion gene of soluble human VEGF receptor KDR-3N with human antibody Fc region

A vector was prepared in the following manner for use in the expression of a fusion protein composed of a soluble human VEGF receptor KDR fragment which corresponds to the 19 amino acids described in SEQ ID NO:35 constituting the signal peptide of human VEGF receptor KDR, the amino acid sequence of the 1st to 294th positions described in SEQ ID NO:34 as mature human VEGF receptor KDR, a linker consisting of 6 amino acid residues (linker #1) and 227 amino acids that constitute a human antibody Fc region (hereinafter referred to as "soluble human VEGF receptor KDR-3N-Fc"). The soluble human VEGF receptor KDR-3N-Fc corresponds to a fusion protein which is composed of three immunoglobulin-like domains from the N-terminal side of the extracellular region of soluble human VEGF receptor KDR, a linker consisting of 6 amino acid residues (linker #1) and a human antibody Fc region.

An *Eco*RI/*Eco*T141 (1.2 kbp) fragment of pUC-KDR-Xb (see Preparation of antigen (1)) and synthetic linkers having the nucleotide sequences of SEQ ID NO:18 and SEQ ID NO:19 were inserted into the *Eco*RI/*Not*I site of pBluescriptII SK(-) to construct pBS-KDR-3N. An *Xba*I-*Sna*BI (1.2 kbp) fragment of the pBS-KDR-3N and a human antibody Fc region-encoding *Sna*BI-*Not*I (0.7 kbp) fragment on pAMoPRFc (see Preparation of antigen (1)) were integrated into the downstream 5'-side *Xba*I and 3'-side *Not*I sites of the transcription initiation point of the polyhedrin gene of a baculovirus recombinant pVL1393 plasmid to construct an expression vector pVL-KDR-3N-Fc for a fusion gene of soluble human VEGF receptor KDR-3N with human antibody Fc.

(6) Construction of expression vector for fusion gene of soluble human VEGF receptor KDR-2N with human antibody Fc region

A vector was prepared in the following manner for use in the expression of a fusion protein composed of a soluble human VEGF receptor KDR fragment which corresponds to the 19 amino acids described in SEQ ID NO:35 constituting the signal peptide of human VEGF receptor KDR, the amino acid sequence of the 1st to 194th positions described in SEQ ID NO:34 as mature human VEGF receptor KDR, a linker, consisting of 6 amino acid residues (linker #1) and 227 amino acids that constitute a human antibody Fc region (hereinafter referred to as "soluble human VEGF receptor KDR-2N-Fc"). The soluble human VEGF receptor KDR-2N-Fc corresponds to a fusion protein which is composed of two immunoglobulin-like domains from the N-terminal side of the extracellular region of soluble human VEGF receptor KDR, a linker consisting of 6 amino acid residues (linker #1) and a human antibody Fc region.

An *Eco*RI/*Vsp*I (0.9 kbp) fragment of pUC-KDR-Xb (see Preparation of antigen (1)) and synthetic linkers having the nucleotide sequences of SEQ ID NO:20 and SEQ ID NO:21 were inserted into the *Eco*RI/*Not*I site of pBluescriptII SK(-) to construct pBS-KDR-2N. An *Xba*I-*Sna*BI (0.9 kbp) fragment of the pBS-KDR-2N and a human antibody Fc region-encoding *Sna*BI-*Not*I (0.7 kbp) fragment on pAMoPRFc (see Preparation of antigen (1)) were integrated into the downstream 5'-side *Xba*I and 3'-side *Not*I sites of the transcription initiation point of the polyhedrin gene of a baculovirus recombinant pVL1393 plasmid to construct an expression vector pVL-KDR-2N-Fc for a fusion gene of soluble human VEGF receptor KDR-2N with human antibody Fc.

(7) Construction of expression vector for fusion gene of soluble human VEGF receptor KDR-1N with human antibody Fc region

A vector was prepared in the following manner for use in the expression of a fusion protein composed of a soluble human VEGF receptor KDR fragment which corresponds to the 19 amino acids described in SEQ ID NO:35 constituting the signal peptide of human VEGF receptor KDR, the amino acid sequence of the 1st to 104th positions described in SEQ ID NO:34 as mature human VEGF receptor KDR, a linker consisting of 6 amino acid residues (linker #1) and 227 amino acids that constitute a human antibody Fc region (hereinafter referred to as "soluble human VEGF receptor KDR-1N-Fc"). The soluble human VEGF receptor KDR-1N-Fc corresponds to a fusion protein which is composed of one immunoglobulin-like domains from the N-terminal side of the extracellular region of soluble human VEGF receptor KDR, a linker consisting of 6 amino acid residues (linker #1) and a human antibody Fc region.

A *Bgl*II/*Not*I (2.8 kbp) fragment of pBS-KDR-2N (see Preparation of antigen (6)) was ligated with synthetic linkers having the nucleotide sequences of SEQ ID NO:22 and SEQ ID NO:23 to construct pBS-KDR-1N. An *Xba*I-*Sna*BI (0.4 kbp) fragment of the pBS-KDR-1N and a human antibody Fc region-encoding *Sna*BI/*Not*I (0.7 kbp) fragment on pAMoAPRFc (see Preparation of antigen (1)) were integrated into the downstream 5'-side *Xba*I and 3'-side *Not*I sites of the transcription initiation point of the polyhedrin gene of a baculovirus recombinant pVL1393 plasmid to constructing an expression vector pVL-KDR-1N-Fc for a fusion gene of soluble human VEGF receptor KDR-1N with human antibody Fc.

(8) Construction of expression vector for fusion gene of soluble human VEGF receptor KDR-7D1N with human antibody Fc region

A vector was prepared in the following manner for use in the expression of a fusion protein composed of a KDR fragment in which a total of 72 amino acids of the 31st amino acid to the 102nd amino acid, that form the first immunoglobulin-like domain from the N-terminal side, were deleted from the soluble human VEGF receptor KDR-7N-Fc (see Preparation of antigen (1)), a linker consisting of 6 amino acid residues (linker #1) and 227 amino acids that constitute a human antibody Fc region (hereinafter referred to as "soluble human VEGF receptor KDR-7Δ1N-Fc"). The soluble human VEGF receptor KDR-7D1N-Fc corresponds to a fusion protein which is composed of the 2nd to 7th immunoglobulin-like domains from the N-terminal side of the extracellular region of soluble human VEGF receptor KDR, a linker consisting of 6 amino acid residues (linker #1) and a human antibody Fc region.

A 2.5 unit portion of Taq polymerase was added to 100 μl of 10 mM MgCl$_2$ 0.001% (W/V) gelatin solution containing 10 pmol of primers having the nucleotide sequences shown in SEQ ID NO:24 and SEQ ID NO:25, 10 ng of the pVL-KDR-7N (see Preparation of antigen (14)) DNA and 10 mM of deoxynucleotide triphosphates. The reaction was carried out as pretreatment at 95°C for 5 minutes and then 30 cycles of polymerase chain reaction (PCR) were repeated, each cycle consisting of 95°C for 90 seconds, 50°C for 90 seconds and finally 72°C for 90 seconds to recover a DNA fragment. The DNA fragment was digested with XbaI and BglII to obtain a 0.8 kbp XbaI/BglII fragment. The DNA fragment and a BglII/NotI (1.6 kbp) fragment of the pVL-KDR-5N (see Preparation of antigen (17)) were inserted into the XbaI/NotI of pBluescriptII SK(-) to prepare pBS-KDR-5Δ1N. An XbaI/HincII (1.6 kbp) fragment of the pBS-KDR-5Δ1N and HincII/NotI (1.2 kbp) fragment of pVL-KDR-7N-Fc (see Preparation of antigen (1)) were integrated into the downstream 5'-side XbaI and 3'-side NotI sites of the transcription initiation point of the polyhedrin gene of a baculovirus recombinant pVL1393 plasmid to construct an expression vector pVL-KDR-7Δ1N-Fc for a fusion gene of soluble human VEGF receptor KDR-7D1N with human antibody Fc region.

(9) Construction of expression vector for fusion gene of soluble human VEGF receptor KDR-5Δ1N with human antibody Fc region

A vector was prepared in the following manner for use in the expression of a fusion protein composed of a KDR fragment in which a total of 72 amino acids of the 31st amino acid to the 102nd amino acid, that form the first immunoglobulin-like domain from the N-terminal side, were deleted from the soluble human VEGF receptor KDR-5N-Fc (see Preparation of antigen (3)), a linker consisting of 6 amino acid residues (linker #1) and 227 amino acids that constitute a human antibody Fc region (hereinafter referred to as "soluble human VEGF receptor KDR-5Δ1N-Fc"). The soluble human VEGF receptor KDR-5Δ1N-Fc corresponds to a fusion protein which is composed of the 2nd to 5th immunoglobulin-like domains from the N-terminal side of the extracellular region of soluble human VEGF receptor KDR, a linker consisting of 6 amino acid residues (linker #1) and a human antibody Fc region.

An XbaI/NotI (1.4 kbp) fragment of pBS-KDR-5D1N (see Preparation of antigen (8)) and a human antibody Fc region-encoding SnaBI-NotI (0.7 kbp) on pAMoAPRFc (see Preparation of antigen (1)) were integrated into the downstream 5'-side XbaI and 3'-side NotI sites of the transcription initiation point of the polyhedrin gene of a baculovirus recombinant pVL1393 plasmid to construct an expression vector pVL-KDR-5Δ1N-Fc for a fusion gene of soluble human VEGF receptor KDR-5Δ1N with human antibody Fc.

(10) Construction of expression vector for fusion gene of soluble human VEGF receptor KDR-4Δ1N with human antibody Fc region

A vector was prepared in the following manner for use in the expression of a fusion protein composed of a KDR fragment in which a total of 72 amino acids of the 31st amino acid to the 102nd amino acid, that form the first immunoglobulin-like domain from the N-terminal side, were deleted from the soluble human VEGF receptor KDR-4N-Fc (see Preparation of antigen (4)), a linker consisting of 6 amino acid residues (linker #2) and 227 amino acids that constitute a human antibody Fc region (hereinafter referred to as "soluble human VEGF receptor KDR-4D1N-Fc"). The soluble human VEGF receptor KDR-4Δ1N-Fc corresponds to a fusion protein which is composed of the 2nd to 4th immunoglobulin-like domains from the N-terminal side of the extracellular region of soluble human VEGF receptor KDR, a linker consisting of 2 amino acid residues (linker #2) and a human antibody Fc region.

The XbaI/BglII-PCR fragment (0.8 kbp) recovered in the Preparation of antigen (8) and a BglII/NotI (0.9 kbp) fragment of pVL-KDR-4N (see Preparation of antigen (18)) were inserted into the XbaI/NotI site of pBluescriptII SK(-) to construct pBS-KDR-4Δ1N. An XbaI-KpnI (1.0 kbp) fragment of the pBS-KDR-4Δ1N and a human antibody Fc region-encoding SnaBI-NotI (0.7 kbp) fragment on pAMoAPRFc (see Preparation of antigen (1)) were integrated into the downstream 5'-side *Xba*I and 3'-side *Not*I sites of the transcription initiation point of the polyhedrin gene of a baculovirus recombinant pVL1393 plasmid to construct an expression vector pVL-KDR-4Δ1N-Fc for a fusion gene of soluble human VEGF receptor KDR-4Δ1N with human antibody Fc.

(11) Construction of soluble human VEGF receptor KDR-7N expression vector

A vector was prepared in the following manner for use in the expression of a soluble human VEGF receptor KDR fragment which corresponds to the 19 amino acids described in SEQ ID NO:35 constituting the signal peptide of human VEGF receptor KDR, the amino acid sequence of the 1st to 738th positions described in SEQ ID NO:34 as mature human VEGF receptor KDR (hereinafter referred to as "soluble human VEGF receptor KDR-7N") and two amino acid residues derived from a linker. The soluble human VEGF receptor KDR-7N corresponds to seven immunoglobulin-like domains from the N-terminal side of the extracellular region of soluble human VEGF receptor KDR.

The pBS-KDR-Xb-S (see Preparation of antigen (1)) was digested with *Sna*BI/*Bam*HI, and synthetic linkers (SEQ ID NO:26 and SEQ ID NO:27) containing a termination codon and *Not*I site were integrated to prepare pBS-KDR(Xb)-S-N. An *Xba*I-*Not*I (2.3 kb) fragment of the pBS-KDR-Xb-S-N was integrated into the downstream 5'-side *Xba*I and 3'-side *Not*I sites of the transcription initiation point of the polyhedrin gene of a baculovirus recombinant pVL1393 plasmid to construct constructing a soluble human VEGF receptor KDR-7N expression vector pVL-KDR-7N.

(12) Construction of soluble human VEGF receptor KDR-7N' expression vector

A vector was prepared in the following manner for use in the expression of a soluble human VEGF receptor KDR fragment which corresponds to the 19 amino acids described in SEQ ID NO:35 constituting the signal peptide of human VEGF receptor KDR and the amino acid sequence of the 1st to 714th positions described in SEQ ID NO:34 as mature human VEGF receptor KDR (hereinafter referred to as "soluble human VEGF receptor KDR-7N'"). The soluble human VEGF receptor KDR-7N' corresponds to a sequence of the N-terminal side to about 2/3 of the seventh immunoglobulin-like domain of the extracellular region of soluble human VEGF receptor KDR.

The pUC-KDR-Xb was digested with *Stu*I and *Sph*I, and synthetic linkers (SEQ ID NO:31 and SEQ ID NO:29) containing a termination codon and *Not*I site were inserted. An *Xba*I-*Not*I (2.2 kbp) fragment was integrated into the downstream 5'-side *Xba*I and 3'-side *Not*I sites of the transcription initiation point of the polyhedrin gene of a baculovirus recombinant pVL1393 plasmid to construct a soluble human VEGF receptor KDR-7N' expression vector pVL-KDR-7N'.

(13) Construction of soluble human VEGF receptor KDR-5N expression vector

A vector was prepared in the following manner for use in the expression of a soluble human VEGF receptor KDR fragment which corresponds to the 19 amino acids described in SEQ ID NO:35 constituting the signal peptide of human VEGF receptor KDR and the amino acid sequence of the 1st to 518th positions described in SEQ ID NO:34 as mature human VEGF receptor KDR (hereinafter referred to as "soluble human VEGF receptor KDR-5N"). The soluble human VEGF receptor KDR-5N corresponds to five immunoglobulin-like domains from the N-terminal side of the extracellular region of soluble human VEGF receptor KDR.

An *Eco*RI/*Hinc*II (1.9 kb) fragment of pUC-KDR-Xb and synthetic DNA (SEQ ID NO:30 and SEQ ID NO:31) containing an *Sna*BI, a termination codon and a *Not*I site were inserted into the *Eco*RI/*Not*I site of pBluescriptII SK(-) to construct pBS-KDR-5N. An *Xba*I-*Not*I (1.6 kb) fragment of the pBS-KDR-5N was integrated into the downstream 5'-side *Xba*I and 3'-side *Not*I sites of the transcription initiation point of the polyhedrin gene of a baculovirus recombinant pVL1393 plasmid to construct a soluble human VEGF receptor KDR-5N expression vector pVL-KDR-5N.

(14) Construction of soluble human VEGF receptor KDR-4N expression vector

A vector was prepared in the following manner for use in the expression of a soluble human VEGF receptor KDR fragment which corresponds to the 19 amino acids described in SEQ ID NO:35 constituting the signal peptide of human VEGF receptor KDR, the amino acid sequence of the 1st to 393rd positions described in SEQ ID NO:34 as mature human VEGF receptor KDR (hereinafter referred to as "soluble human VEGF receptor KDR-4N") and two amino acid residues derived from a linker. The soluble human VEGF receptor KDR-4N corresponds to four immunoglobulin-like domains from the N-terminal side of the extracellular region of soluble human VEGF receptor KDR.

An *Xba*I-*Kpn*I (1.2 kb) fragment of the pAMo-4N-Fc (see Preparation of antigen (4)) and synthetic linkers having the nucleotide sequences of SEQ ID NO:32 and SEQ ID NO:33 were integrated into the downstream 5'-side *Xba*I and 3'-side *Not*I sites of the transcription initiation point of the polyhedrin gene of a baculovirus recombinant pVL1393 plasmid to construct a soluble human VEGF receptor KDR-4N expression vector pVL-KDR-4N.

(15) Construction of soluble human VEGF receptor KDR-3N expression vector

A vector was prepared in the following manner for use in the expression of a soluble human VEGF receptor KDR fragment which corresponds to the 19 amino acids described in SEQ ID NO:35 constituting the signal peptide of human VEGF receptor KDR, the amino acid sequence of the 1st to 294th positions described in SEQ ID NO:34 as mature human VEGF receptor KDR (hereinafter referred to as "soluble human VEGF receptor KDR-3N") and two amino acid residues derived from a linker. The soluble human VEGF receptor KDR-3N corresponds to three immunoglobulin-like domains from the N-terminal side of the extracellular region of soluble human VEGF receptor KDR.
An *Xba*I-*Sna*BI (1.2 kbp) fragment of the pBS-KDR-3N (see Preparation of antigen (5)) and synthetic linkers having the nucleotide sequences of SEQ ID NO:26 and SEQ ID NO:27 were integrated into the downstream 5'-side *Xba*I and 3'-side *Bgl*II sites of the transcription initiation point of the polyhedrin gene of a baculovirus recombinant pVL1393 plasmid to construct a soluble human VEGF receptor KDR-3N expression vector pVL-KDR-3N.

(16) Construction of soluble human VEGF receptor KDR-7$\Delta$1N expression vector

A vector was prepared in the following manner for use in the expression of a fusion protein composed of a KDR fragment in which a total of 72 amino acids of the 31st amino acid to the 102nd amino acid, that form the first immunoglobulin-like domain from the N-terminal side, were deleted from the soluble human VEGF receptor KDR-7N (see Preparation of antigen (14)), a linker consisting of 6 amino acid residues (linker #1) and 227 amino acids that constitute a human antibody Fc region (hereinafter referred to as "soluble human VEGF receptor KDR-7$\Delta$1N"). The soluble human VEGF receptor KDR-7$\Delta$1N corresponds to a fusion protein which is composed of the 2nd to 7th immunoglobulin-like domains from the N-terminal side of the extracellular region of soluble human VEGF receptor KDR and a linker consisting of 6 amino acid residues (linker #1).
An *Xba*I/*Hin*cII (1.6 kbp) fragment of the pBS-KDR-5$\Delta$1N (see Preparation of antigen (9)) and a *Hin*cII/*Not*I (0.67 kbp) fragment of the pVL-KDR-7N (see Preparation of antigen (14)) were integrated into the downstream 5'-side *Xba*I and 3'-side *Not*I sites of the transcription initiation point of the polyhedrin gene of a baculovirus recombinant pVL1393 plasmid to construct a soluble human VEGF receptor KDR-7D1N expression vector pVL-KDR-7D1N.

(17) Preparation of a recombinant virus for carrying out expression of soluble human VEGF receptor KDR in insect cells

In order to produce a protein by insect cells, it is necessary to prepare a recombinant virus into which a gene of interest has been integrated, and the preparation includes a step in which cDNA coding for the protein of interest is integrated into a special plasmid, which is called transfer vector, and a step in which co-transfection of an insect cell with a wild type virus and the transfer vector is carried out to obtain a recombinant virus by homologous recombination. These steps were carried out in the following manner using BaculoGold Starter Kit manufactured by Pharmingen (production number PM-21001K) in accordance with the manual attached thereto.
A recombinant baculavirus was prepared in the following wanner by introducing a filamentous baculovirus DNA (BaculoDold baculovirus DNA, manufactured by Pharmingen) and the prepared transfer vector DNA into an insect cell Sf9 (manufactured by Pharmingen) cultured using TMN-FH Insect Medium (manufactured by Pharmingen), by lipofection [*Protein, Nucleic acid and Enzyme, 37*: 2701 (1992)].
A 1 µg portion of the expression vector prepared in (1) and 20 ng of the filamentous baculavirus DNA were dissolved in 12 µl of distilled water to which was further added a mixture of 6 µl of lipofectin and 6 µl of distilled water, and the resulting mixture was allowed to stand at room temperature for 15 minutes. Separately, $1\times10^6$ of Sf9 cells were suspended in 2 ml of Sf900-II medium (manufactured by Gibco) and put into a 35 mm diameter plastic dish for cell culture use. To this was added entire volume of the above mixed solution of plasmid DNA, filamentous baculovirus DNA and lipofectin, and the cells were cultured at 27°C for 3 days and then 1 ml of the culture supernatant containing recombinant virus was collected. The dish was supplemented with 1 ml of fresh Sf900-II medium and again cultured at 27°C for 3 days to further obtain 1.5 ml of the culture supernatant containing the recombinant virus. The same procedure was repeated using each of the expression vectors prepared in (2) to (16).
Next, each of the thus obtained recombinant viruses for use in the protein expression was propagated in the following manner.
A total of $2\times10^7$ Sf9 cells were suspended in 10 ml of Sf900-II medium, put into a 175 cm$^2$ flask (manufactured by Greiner) and allowed to stand at room temperature for 1 hour to adhere cells to the flask. After the standing, the supernatant was discarded, and 15 ml of TMN-FH insect medium and a 1 ml portion of the above culture supernatant containing recombinant virus were added to carry out 3 days of culturing at 27°C. After the culturing, the supernatant was centrifuged at 1,500 $\times$ g for 10 minutes to remove the cells to obtain a recombinant virus solution used in the protein expression.
Titer of the virus in the thus obtained recombinant virus solution was calculated by the method described in BaculoGold Starter Kit Manual (manufactured by Pharmingen).

A total of $6\times10^6$ Sf9 cells were suspended in 4 ml of Sf900-II medium, put into a 60 mm diameter plastic dish for cell culture use and then allowed to stand at room temperature for 1 hour to adhere cells to the dish. Next, the supernatant was discarded, 400 μl of Sf900-II medium and the above recombinant virus solution which had been diluted 1,000 times with Sf900-II medium were added thereto and allowed to stand at room temperature for 1 hour, and then the medium was removed, and a medium (prepared by mixing 1 ml of sterilized 5% Agarplaque plus agarose aqueous solution with 4 ml of TMN-FH insect medium and maintained at 42°C) containing 5 ml of 1% low melting point agarose (Agarplaque Aqarose, manufactured by Pharmingen) was poured into the dish. After standing at room temperature for 15 minutes, the dish was sealed with a vinyl tape to prevent drying and put into a sealable plastic container to carry out 6 days of culturing at 27°C. A 1 ml portion of PBS containing 0.01% of Neutral Red was added to the dish to carry out additional 1 day of the culturing, and then the number of formed plaques was counted. By the above operation, it was found that each of the recombinant virus solutions contained about $1\times10^7$ plaque forming units (hereinafter referred to as "PFU")/ml of the virus.

(18) Expression of various derivatives of soluble human VEGF receptor KDR-Fc and various derivatives of soluble human VEGF receptor KDR in insect cells, and their purification

The various derivatives of the soluble human VEGF receptor KDR-Fc and various derivatives of the soluble human VEGF receptor KDR shown in 1(1) to (16) were obtained in the following manner. A total of $4\times10^7$ High Five cells were suspended in 30 ml of EX-CELL™ 400 medium (manufactured by JRH Bioscience) contained in a 175 cm² flask (manufactured by Greiner) and allowed to stand at room temperature for 1 hour to adhere them to the flask. A 1 ml portion of a solution containing the recombinant viruses derived from each of the transfer vectors obtained in 1(1) to (16) at a concentration of about $1\times10^8$ to $3\times10^8$ PFU/ml was added thereto to carry out 2 hours of infection at room temperature. The culture supernatant was discarded and 30 ml of fresh EX-CELL™ 400 was added, and the culturing was carried out at 27°C for 3 to 4 days. After completion of the culturing, the culture supernatant was recovered and centrifuged at $1,500 \times g$ for 10 minutes to obtain a supernatant.

Various derivatives of the soluble human VEGF receptor KDR-Fc were purified in the following manner using a ProSep A column.

A column was packed with about 1 ml of ProSep A (manufactured by Bioprocessing), and the column was washed using 10 ml of 20 mM sodium phosphate buffer (pH 7.2) at a flow rate of 1 ml/min. After the washing, 500 to 1,000 ml of the culture medium containing the soluble human VEGF receptor KDR prepared in the above-described manner was passed through the ProSep A column at a flow rate of 100 ml/hour. After further washing using 10 ml of 20 mM sodium phosphate buffer (pH 7.2) at a flow rate of 1 ml/min, 7 ml of 50 mM citrate buffer (pH 3) was passed through the column to carry out elution of proteins absorbed to the ProSep A column. The proteins contained in each fraction was analyzed by SDS polyacrylamide gel electrophoresis (SDS-PAGE).

Various derivatives of the soluble human VEGF receptor KDR were purified in the following manner.

A column packed with 50 ml of DEAE-Sepharose CL-6B (manufactured by Pharmacia Biotech) and a column packed with 40 ml of Heparin Sepharose CL-6B (manufactured by Pharmacia Biotech) were connected in series, the former column on the inlet side and the latter on the outlet side, and washed with 300 ml of 20 mM sodium phosphate buffer (pH 8). After the washing, 400 to 800 ml of the culture medium containing soluble human VEGF receptor KDR was passed through the columns at a flow rate of 50 to 100 ml/hour. After further washing with 300 ml of 20 mM sodium phosphate buffer (pH 8), 400 ml of 0 to 1 M NaCl/20 mM sodium phosphate buffer was passed only through the Heparin Sepharose CL-6B column with continuous density gradient to carry out elution of the absorbed proteins. The eluate was fractionated in 7 ml portions and the proteins contained in each fraction was analyzed by SDS-PAGE to recover 60 to 80 ml of fractions containing the soluble human VEGF receptor KDR. The thus recovered purified fractions were concentrated using CentriPrep 10 (manufactured by Amicon) to obtain soluble human KDR3N, KDR4N, KDR5N, KDR7N' and KDR7N as solutions of 2.8 ml, 8 ml, 5.5 ml, 4 ml and 4.8 ml, respectively, (protein concentration/purity values were 345.5 μg/ml/30%, 264 μg/ml/50 to 60%, 380.5 μg/ml/70%, 1.59 mg/ml/60% and 815 μg/ml/70 to 80%).

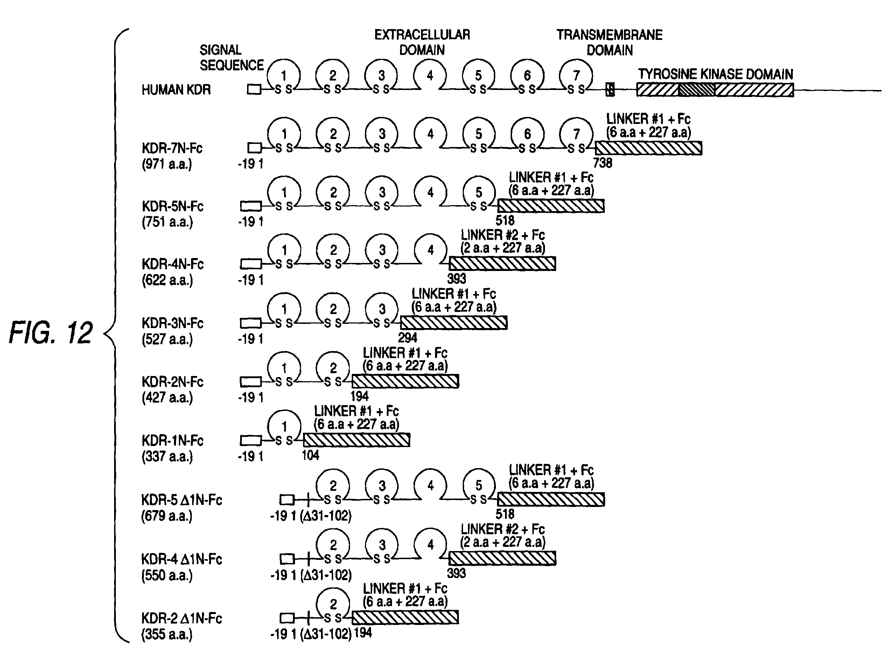

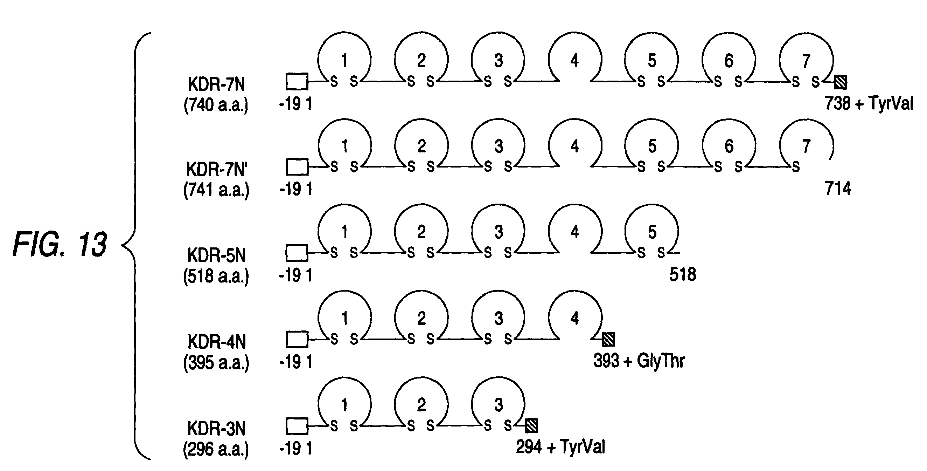

Schematic illustrations of the thus obtained various derivatives of the soluble human VEGF receptor KDR-Fc and various derivatives of the soluble human VEGF receptor KDR are shown in Fig. 12 and Fig. 13.

(19) Confirmation of purity of soluble human VEGF receptor KDR

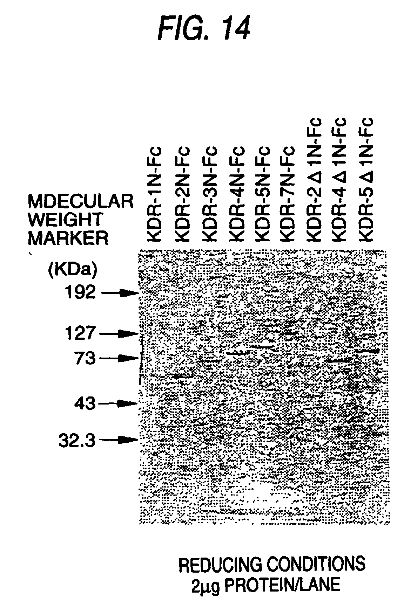

Purity of the purified soluble human VEGF receptor KDR-Fc was confirmed using SDS-PACE. The SDS-PAGE was carried out in accordance with a method described in a literature [*Anticancer Research, 12*: 1121 (1992)]. A 5 to 20% gradient gel (manufactured by Atto) was used as the gel, and each of the KDR-Fc derivatives in an amount of 2 μg as protein per lane was subjected to the electrophoresis under reducing condition and stained with Coomassie Brilliant Blue. The results are shown in Fig. 14. The purity of KDR-7N-Fc, KDR-5N-Fc, KDR-4N-Fc, KDR-3N-Fc, KDR-2N-Fc, KDR-1N-Fc, KDR-5Δ1N-Fc and KDR-4Δ1N-Fc was 95% or more.

(20) Purification of control antigen protein

The control antigen protein was obtained in the following manner. A total of $4\times10^7$ High Five cells were suspended in 30 ml of EX-CELL™ 400 medium (manufactured by JRH Bioscience) contained in a 175 cm² flask (manufactured by Greiner), allowed to stand at room temperature for 1 hour to adhere them to the flask and then cultured at 27°C for 3 to 4 days. After completion of the culturing, the culture supernatant was recovered and centrifuged at 1,500 × g for 10 minutes to obtain a supernatant.
A column was packed with about 20 ml of Heparin-Sepharose CL-6B Gel (manufactured by Pharmacia Biotech AB) and washed using 200 ml of 20 mM Tris-HCl (pH 7.5) buffer at a flow rate of 0.5 ml/min. After the washing, 500 ml of the High Five cell culture medium prepared in the above was passed through the Heparin-Sepharose CL-6B column at a flow rate of 0.5 ml/min. After further washing with 200 ml of 20 mM Tris-HCl (pH 7.5) containing 0.2 M NaCl at a flow rate of 0.5 ml/min, 200 ml of a buffer comprised of 20 mM Tris-HCl (pH 7.5) containing 1 M NaCl was passed through the column to carry out elution of proteins absorbed to the Heparin-Sepharose. The 1 M NaCl elution fractions were concentrated using CentriPrep 10 (manufactured by Amicon) to obtain the control antigen protein as 7 ml of solution having a protein concentration of 867 μg/ml.

(21) Confirmation of human VEGF binding activity of various derivatives of the soluble human VEGF receptor KDR-Fc

The human VEGF binding activity of various soluble human VEGF receptor KDR-Fc derivatives (KDR-7N-Fc, KDR-5N-Fc, KDR-4N-Fc, KDR-3N-Fc, KDR-2N-Fc, KDR-1N-Fc, KDR-5Δ1N-Fc, KDR-4Δ1N-Fc and KDR-2Δ1N-Fc) obtained in (18) was confirmed by the following VEGF binding inhibition test (21-1) and VEGF binding test (21-2).

(21-1) VEGF binding inhibition test

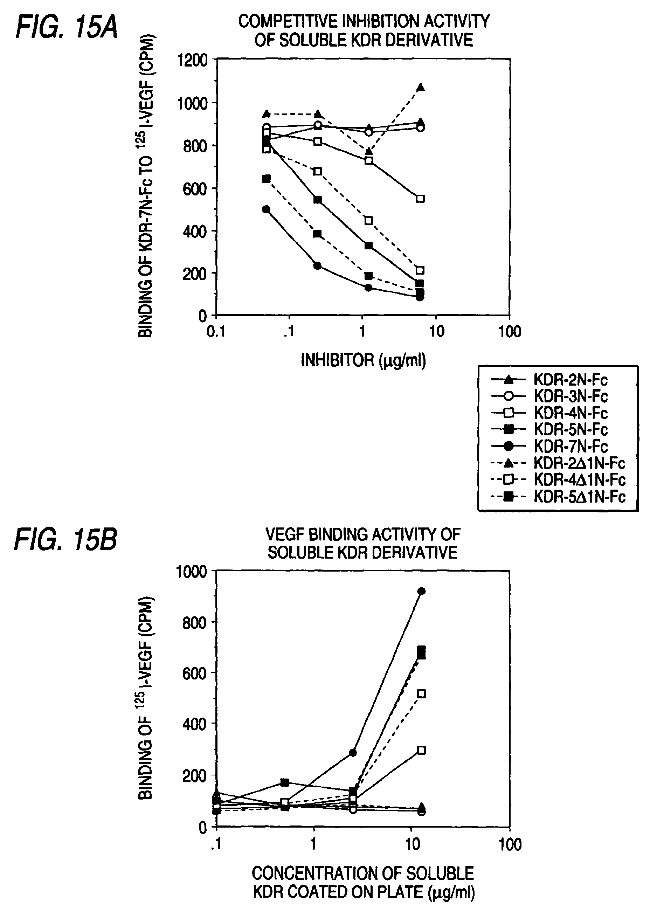

Methanol was dispensed in 100 μl portions into wells of a 96-well Immobilon™-P Filtration Plate (manufactured by Millipore) to give hydrophilic nature to the PVDF membrane on the bottom of the plate. After washing with water, the soluble human KDR-7N-Fc diluted to 4 μg/ml with PBS was dispensed in 50 μl/well portions and allowed to stand overnight at 4°C for absorption. After washing, PBS containing 1% bovine serum albumin (BSA) was added in 200 μl/well portions and the reaction was carried out at room temperature for 30 minutes to block the remaining active residues. After washing with PBS, each of the purified soluble human VEGF receptor KDR-Fc derivatives (KDR-7N-Fc, KDR-5N-Fc, KDR-4N-Fc, KDR-3N-Fc, KDR-2N-Fc, KDR-1N-Fc, KDR-5Δ1N-Fc, KDR-4Δ1N-Fc and KDR-2Δ1N-Fc) obtained in (18) was dispensed in 50 μl/well portions (final concentration of 0.05 to 6.25 ng/ml), $^{125}$I-labeled human VEGF (final concentration 4 ng/ml; manufactured by Amersham) was dispensed in 50 μl/well portions and then the reaction was carried out at room temperature for 1.5 hours. After washing with 0.05% Tween-PBS, the wells were dried at 50°C, Microscinti-O (manufactured by Packard) was added in 10 μl/well portions and then radioactivity of the $^{125}$I-labeled human VEGF bound to each well was measured using Top Count (manufactured by Packard).
The results are shown in Fig. 15A. It was shown that KDR-7N-Fc, KDR-5Δ1N-Fc, KDR-5N-Fc, KDR-4Δ1N-Fc and KDR-4N-Fc inhibit binding of the $^{125}$I-labeled human VEGF to the soluble human KDR7N-Fc in a concentration-dependent manner. On the other hand, KDR-3N-Fc, KDR-2N-Fc, KDR-1N-Fc and KDR-2Δ1N-Fc showed no binding inhibition activity. The inhibition activity is shown as follows: KDR-7N-Fc > KDR-5Δ1N-Fc > KDR-5N-Fc > KDR-4Δ1N-Fc > KDR-4N-Fc. Thus, it was shown that at least the 1st, 6th and 7th Ig-like domains from the N-terminal side are not concerned in the binding of VEGF to KDR. It was shown also that a derivative can bind to VEGF when it has the 2nd, 3rd and 4th Ig-like domains (103rd to 393rd amino acids from the N-terminal) from the N-terminal side.

(21-2) VEGF binding test

Methanol was dispensed in 100 μl/well portions into a 96-well Immobilon™-P Filtration Plate (manufactured by Millipore) to give hydrophilic nature to the PVDF membrane on the bottom of the plate. After washing with water, each of the purified soluble human VEGF receptor KDR-Fc derivatives (KDR-7N-Fc, KDR-5N-Fc, KDR-4N-Fc, KDR-3N-Fc, KDR-2N-Fc, KDR-1N-Fc, KDR-5Δ1N-Fc, KDR-4Δ1N-Fc and KDR-2Δ1N-Fc) obtained in (18) diluted to 0.1 to 12.5 μg/ml with PBS was dispensed in 50 μl/well portions and allowed to stand overnight at 4°C for absorption. After washing, PBS containing 1% bovine serum albumin (BSA) was added in 200 μl/well portions and the reaction was carried out at room temperature for 3 minutes to block the remaining active residues. After washing with PBS, $^{125}$I-labeled human VEGF (final concentration 4 ng/ml; manufactured by Amersham) was dispensed in 50 μl/well portions and then the reaction was carried out at room temperature for 1.5 hours. After washing with 0.05% Tween-PBS, the wells were dried at 50°C, Microscinti-O (manufactured by Packard) was added in 10 μl/well portions and then radioactivity of the $^{125}$I-labeled human VEGF bound to each well was measured using Top Count (manufactured by Packard).

The results are shown in Fig. 15B. It was shown that KDR-7N-Fc, KDR-5Δ1N-Fc, KDR-5N-Fc, KDR-4Δ1N-Fc and KDR-4N-Fc bind to the $^{125}$I-labeled human VEGF in a concentration-dependent manner. On the other hand, KDR-3N-Fc, KDR-2N-Fc, KDR-1N-Fc and KDR-2Δ1N-Fc The inhibition activity is shown as follows: KDR-7N-Fc > KDR-5Δ1N-Fc = KDR-5N-Fc > KDR-4Δ1N-Fc > KDR-4N-Fc . Thus, it was shown that at least the 1st, 6th and 7th Ig-like domains from the N-terminal site are not concerned in the binding of VEGF to KDR. It was shown also that a derivative can bind to VEGF when it has the 2nd, 3rd and 4th Ig-like domains (103rd to 393rd amino acids from the N-terminal) from the N-terminal site.

(22) Expression of human VEGF in insect cells

The human VEGF was obtained in the following manner. A total of $4\times10^7$ High Five cells were suspended in 30 ml of EX-CELL™ 400 medium (manufactured by JRH Bioscience) contained in a 175 cm$^2$ flask (manufactured by Greiner) and allowed to stand at room temperature for 1 hour to adhere them to the flask. A 1 ml portion of a solution containing a human VEGF recombinant baculovirus obtained by a method described in a literature [*Cell Growth & Differentiation, 7*: 213 (1996)] at a concentration of about 1 to $3\times10^8$ PFU/ml was added thereto to carry out 2 hours of infection at room temperature. The culture supernatant was discarded and 30 ml of fresh EX-CELL™ 400 medium was added, and the culturing was carried out at 27°C for 3 to 4 days. After completion of the culturing, the culture supernatant was recovered and centrifuged at $1,500 \times g$ for 10 minutes to obtain a supernatant.

A column was packed with about 40 ml of Heparin-Sepharose CL-6B Gel (manufactured by Pharmacia Biotech AB) and washed using 400 ml of 20 mM Tris-HCl (pH 7.5) buffer at a flow rate of 0.5 ml/min. After the washing, 1,500 ml of the culture medium containing the human VEGF prepared in the above-described manner was passed through the Heparin-Sepharose CL-6B column at a flow rate of 0.5 ml/min. After further washing using 400 ml of 20 mM Tris-HCl (pH 7.5) at a flow rate of 0.5 ml/min, 120 ml of buffers comprised of 20 mM Tris-HCl (pH 7.5) containing 0.2 M, 0.5 M and 1 M NaCl were passed through the column in this order to carry out elution of proteins absorbed to the Heparin-Sepharose while fractionating the eluate in 8 ml portions. By analyzing the proteins contained in each fraction by SDS polyacrylamide gel electrophoresis, 120 ml of fractions containing the human VEGF (0.5 to 1 M NaCl fractions) were recovered. After concentration using CentriPrep-10 (manufactured by Amicon), 4 ml of human VEGF solution (protein concentration 1.2 mg/ml) was obtained.

2. Immunization of animal and preparation of antibody producing cells

A 10 to 50 μg portion of each of the antigens obtained in 1(18) was administered, together with 2 mg of aluminum gel and $1\times10^9$ cells of pertussis vaccine (manufactured by Chiba Serum Institute), to five weeks old female BALB/c (manufactured by Japan SLC) or B6C3F1 mice (manufactured by Charles River Japan) or female SD rats (manufactured by Japan SLC), and 2 weeks thereafter, 10 to 50 μg of the protein was administered once a week for a total of 4 weeks. Also, $1\times10^7$ of NIH3T3-KDR cells were administered to 3 female BALB/c (manufactured by Japan SLC) of 5 weeks of age for a total of 6 times. A blood sample of each animal was collected from the venous plexus of the fundus of the eye, the heart or the caudal vein, its serum antibody titer was examined by an enzyme immunoassay shown in the following, and the spleen of each mouse or rat which showed sufficient antibody titer was excised 3 days after the final immunization. In this case, the five weeks old female BALB/c mice to which the NIH3T3-KDR cells were administered were not immunized, and the titer for soluble KDR did not increase.

The spleen was cut to pieces in MEM medium (manufactured by Nissui Pharmaceutical), the cells were unbound using a pair of forceps and centrifuged (1,200 rpm, 5 minutes), the supernatant was discarded and then the sediment was treated with Tris-ammonium chloride buffer (pH 7.65) for 1 to 2 minutes for removing erythrocytes, washed three times with MEM medium and used for cell fusion.

3. Enzyme Immunoassay

The antiserum derived from a mouse or rat immunized with each of the soluble human VEGF receptor KDR-Fc derivatives and KDR derivatives obtained in 1(18) and of the culture supernatant of a hybridoma was measured using the soluble human VEGF receptor KDR-Fc derivatives and KDR derivatives obtained from the insect cell culture supernatants of 1(18) as the antigen. Each of the soluble human VEGF receptor KDR-Fc derivatives and KDR derivatives, the heparin column absorption fraction of High Five cell culture supernatant obtained in 1(20) as a control antigen or an anti-GD3 mouse human chimera antibody KM871 [*Cancer Immunology and Immunotherapy, 36*: 373 (1993)] was diluted to 1 to 10 μg/ml with PBS and dispensed in 50 μl/well portions into a 96 well plate for EIA use (manufactured by Greiner) and allowed to stand overnight at 4°C for absorption. After washing, PBS containing 1% bovine serum albumin (BSA) was added in 100 μl/well portions and 1 hour of the reaction was carried out at room temperature to block the remaining active residues. The 1% BSA-PBS was discarded, and antiserum of an immunized mouse or immunized rat and culture supernatant of a hybridoma were dispensed in 50 μl/well portions to carry out 2 hours of the reaction. After washing with 0.05% Tween-PBS, peroxidase-labeled rabbit anti-mouse immunoglobulin or peroxidase-labeled rabbit anti-rat immunoglobulin (both manufactured by DAKO) was dispensed in 50 μl/well portions to carry out 1 hour of the reaction at room temperature and then, after washing with 0.05% Tween-PBS, color development was carried out using an ABTS substrate solution [ammonium 2,2-azinobis(3-ethylbenzothiazole-6-sulfonate)] and its absorbance Emax (manufactured by Molecular Devices) at OD415 nm was measured.

4. Preparation of mouse myeloma cells

By culturing an 8-azaguanine-resistant mouse myeloma cell line P3-U1 using a normal medium, $2 \times 10^7$ or more of its cells were prepared and used as the parent line in cell fusion.

5. Preparation of hybridoma

The mouse splenocytes or rat splenocytes obtained in 2 and the myeloma cells obtained in 4 were mixed at a ratio of 10:1 and centrifuged (1,200 rpm, 5 minutes) to discard the supernatant, and the thus precipitated cells were thoroughly unbound, to which, while stirring at 37°C, were added 2 g of polyethylene glycol 1000 (PEG-1000), 0.2 to 1 ml/$10^8$ mouse splenocytes of a mixed solution of 2 ml of MEM medium and 0.7 ml of DMSO, 1 to 2 ml of the MEM medium several times at an interval of 1 to 2 minutes and then the MEM medium to adjust the total volume to 50 ml. After centrifugation (900 rpm, 5 minutes), the supernatant was discarded and the cells were gently unbound and then suspended in 100 ml of HAT medium by gently drawing up into and discharging from a measuring pipette.

6. Screening of hybridoma by binding ELISA

The suspension obtained in 5 was dispensed in 100 μl/well portions into a 96 well culture plate and cultured in an atmosphere of 5% $CO_2$ at 37°C for 10 to 14 days in a 5% $CO_2$ incubator. Each of the culture supernatants were examined by the enzyme immunoassay described in 3 of Reference Example 2 to select wells which specifically reacted with the soluble human VEGF receptor KDR-Fc derivatives and KDR derivatives obtained in 1(18) of Reference Example 2 but did not react with the control antigen obtained in 1(20), and their cloning was repeated twice by further changing to the HT medium and normal medium to establish hybridomas capable of producing the anti-human VEGF receptor KDR monoclonal antibody. The results are shown below.

**Table 4**

| Animal used | Number of animals | Immunogen | Screening source | Screening Method | Number of wells screened | Number of hybridomas established |
|---|---|---|---|---|---|---|
| SD rat | 3 | KDR(7N') | KDR(7N') | Binding ELISA | 3024 | 4 (KM1660-1663) |
| SD rat | 2 | KDR(7N') | KDR(7N) | | 2016 | 1 (KM1667) |
| Balb/c mouse | 1 | KDR(2N)-Fc | KDR(7N) | | 420 | 7 (KM1859-1865) |
| Balb/c mouse | 1 | KDR/NIH3T3 cell | KDR(7N') | | 504 | 1 (KM1659) |
| Balb/c mouse | 1 | KDR(7N') | KDR(7N') | | 420 | 1 (KM1664) |
| Balb/c mouse | 1 | KDR(7N') | KDR(7N')-Fc | | 420 | 2 (KM1665, 1666) |
| Balb/c mouse | 2 | KDR(2N)-Fc | KDR(2N)-Fc | | 840 | 1 (KM1668) |
| Balb/c mouse | 2 | KDR(7N') | KDR(7N) | | 840 | 1 (KM1768) |
| Balb/c mouse | 2 | KDR(7N) | KDR(3N)-Fc | | 840 | 2 (KM1825, 1826) |
| Balb/c mouse | 2 | KDR(7N) | KDR(5N)-Fc | | 840 | 4 (KM1827-1830) |
| Balb/c mouse | 2 | KDR(7N) | KDR(5N) | | 840 | 14 (KM1831-1838, 1853-1858) |
| Balb/c mouse | 4 | KDR(5N) | KDR(5N) | | 1680 | 10 (KM1943-1950, 1932, 1933) |
| Balb/c mouse | 1 | KDR(7N) | KDR(7N') | | 420 | 3 (KM1778-1780) |
| Balb/c mouse | 1 | KDR(5N) | KDR(5N) | | 504 | 3 (KM1987-1989) |
| Balb/c mouse | 1 | KDR(5N)-Fc | KDR(5N) | | 420 | 1 (KM1942) |
| Balb/c mouse | 3 | KDR(5N)-Fc | KDR(5N) | | 1260 | 8 (KM1943-1950) |
| Balb/c mouse | 3 | KDR(7N)-Fc | KDR(5A1N)-Fc | | 1260 | 11 (KM1965-1975) |
| B6C3F1 mouse | 1 | KDR(7N)-Fc | KDR(5A1N)-Fc/RI | VEGF-KDR binding inhibition test | 420 | 0 |
| B6C3F1 mouse | 1 | KDR(5A1N)-Fc | KDR(7N)-Fc/RI | | 420 | 7 (KM1991-1997) |

Using hybridomas obtained from a total of 32 animals of Balb/c mice, B6C3F1 mice and SD rats which had been immunized with various derivatives of the soluble human VEGF receptor KDR-Fc and KDR obtained in 1(18) and with KDR-NIH3T3 cells, about 16548 wells were screened and a total of 74 clones of anti-human VEGF receptor KDR monoclonal antibodies which reacted specifically with the various derivatives of soluble human VEGF receptor KDR-Fc and KDR obtained in 1(18) but did not react with the control antigen obtained in 1(20) or KM871 were obtained and named as shown in Table 4. Among these anti-human VEGF receptor KDR monoclonal antibodies, 40 monoclonal antibodies (KM1668, 1768, 1825, 1826, 1827, 1828, 1829, 1831, 1835, 1837, 1853, 1856, 1857, 1859, 1860, 1861, 1862, 1863, 1864, 1865, 1933, 1942, 1943, 1944, 1945, 1946, 1947, 1948, 1949, 1950, 1987, 1988, 1989, 1858, 1832, 1833, 1834, 1836, 1838 and 1932) showed their reaction with KDR on the cell surface when measured by the immunocyte staining method. However, it was not able to obtain a monoclonal antibody capable of inhibiting biological activities of KDR, such as an activity of inhibiting growth acceleration activity of vascular endothelial cells by VEGF stimulation.

7. Epitope analysis of monoclonal antibodies

Specificity of the anti-human VEGF receptor KDR monoclonal antibodies described in 6 was confirmed by the enzyme immunoassay described in 3 using hybridoma culture supernatants.

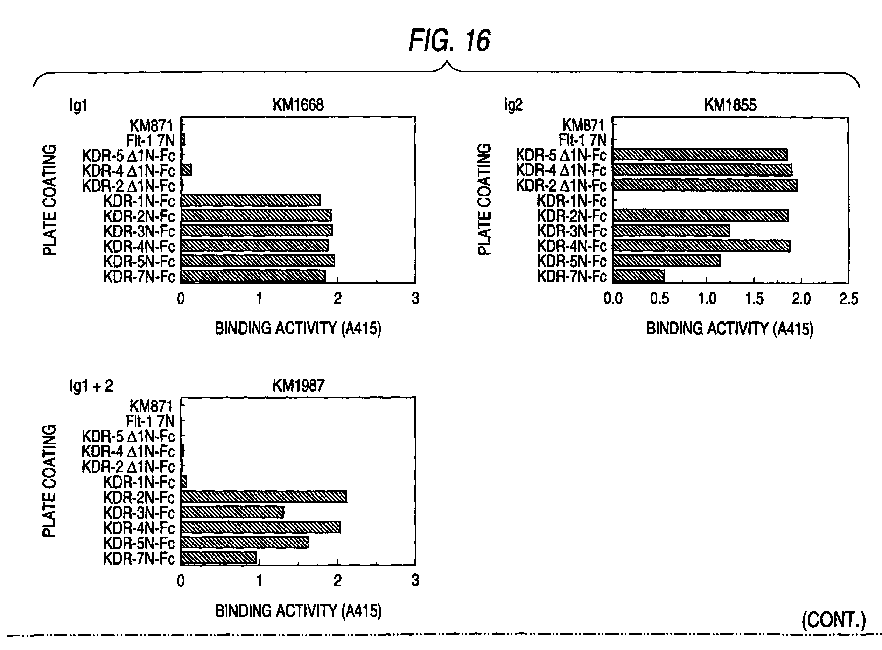

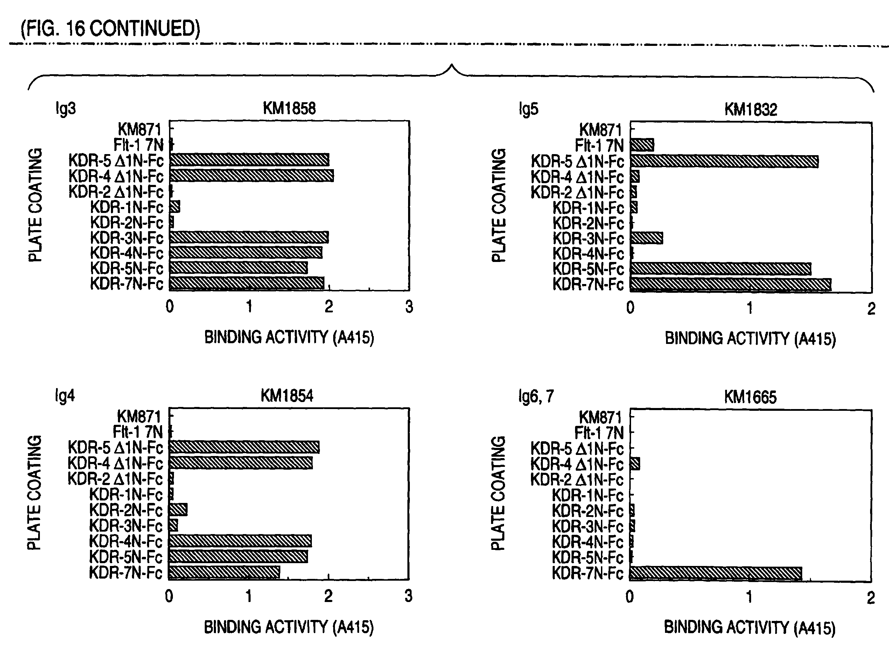

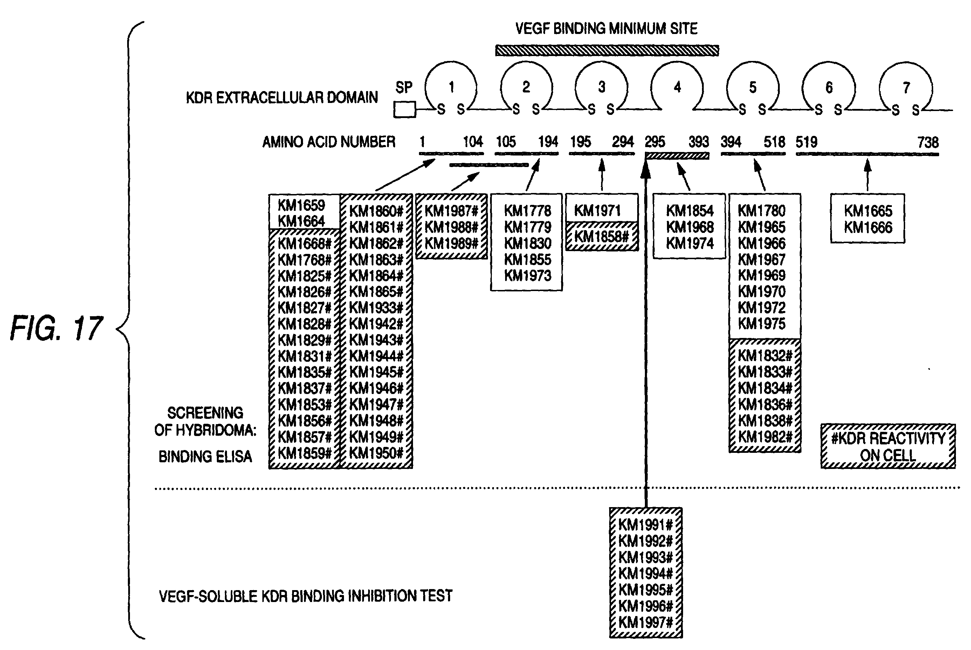

Typical results and summarized results are shown in Fig. 16 and Fig. 17. Among the above 74 monoclonal antibody species, 32 species including KM1668 reacted with the 1st Ig-like domain (corresponds to 1st to 104th amino acids), 3 species including KM1987 reacted with the first Ig-like domain (corresponds to 1st to 104th amino acids) and the second Ig-like domain (corresponds to 105th to 194th amino acids), 5 species including KM1855 reacted with the second Ig-like domain (corresponds to 105th to 194th amino acids), 2 species including KM1858 reacted with the third Ig-like domain (corresponds to 195th to 294th amino acids), 3 species including KM1854 reacted with the fourth Ig-like domain (corresponds to 295th to 393rd amino acids), 14 species including KM1832 reacted with the fifth Ig-like domain (corresponds to 394th to 518th amino acids) and 2 species including KM1665 reacted with the sixth and seventh Ig-like domains (corresponds to 519th to 738th amino acids). Thus, 43% of the monoclonal antibodies having high immunogenicity for the first Ig-like domain reacted with the first Ig-like domain. Although the first Ig-like domain of KDR is not concerned in the binding activity of VEGF as shown in 1(21), it was assumed that a monoclonal antibody showing neutralizing activity is difficult to prepare by ELISA screening due to the high immunogenicity.

8. Measurement of antibody titer by [$^{125}$I]VEGF-KDR binding inhibition assay

In order to exclude monoclonal antibodies for the first Ig-like domain which has high immunogenicity and is not concerned in the neutralizing activity, mice were immunized with KDR-5Δ1N-Fc obtained in 1(18). Binding inhibition activity of human VEGF to human VEGF receptor KDR of mouse antiserum was evaluated in the following manner.

Methanol was dispensed in 100 μl/well portions into a 96-well MultiScreen-IP Plate (manufactured by Millipore) to make PVDF membrane of the plate bottom into hydrophilic state. After washing with water, the soluble human VEGF receptor KDR-7N-Fc diluted to 4 μg/ml with PBS was dispensed in 50 μl/well portions and allowed to stand overnight at 4°C for absorption. After washing, PBS containing 1% bovine serum albumin (BSA) was added in 200 μl/well portions and the reaction was carried out at room temperature for 30 minutes to block the remaining active residues. After washing with PBS, an antiserum diluted 100, 1,000 or 10,000 times with 1% BSA-PBS solution, a purified monoclonal antibody diluted with 1% BSA-PBS solution (0.01 to 25 μg/ml) or a hybridoma culture supernatant was dispensed in 50 μl/well portions, 4 ng/ml of $^{125}$I-labeled human VEGF (manufactured by Amersham) was dispensed in 50 μl/well portions and then the reaction was carried out at room temperature for 1.5 hours.

After washing with 0,05% Tween-PBS, the wells were dried at 50°C, Microscinti-O (manufactured by Packard) was added in 10 μl/well portions and then radioactivity of the $^{125}$I-labeled human VEGF bound to each veil was measured using Top Count (manufactured by Packard).

Results of the measurement of the activity in hybridoma culture supernatants are shown in Table 5.

Table 5

| | | % Inhibition | | |
|---|---|---|---|---|
| | Serum dilution | 1/10,000 | 1/1,000 | 1/100 |
| Immunogen | Mouse # | | | |
| Control | | 0 | 2.6 | 0 |
| KDR7N-Fc | #B | 0.35 | 0 | 47.7 |
| | #C | 16.1 | 6.7 | 58 |
| | #D | 7.8 | 10.5 | 56.1 |
| KDR5N | #A | 9.7 | 3.4 | 16.5 |
| | #C | 14.1 | 0 | 1.1 |
| | #3 | 1 | 0 | 19.2 |
| KDR5N-Fc | #B | 5.7 | 14.7 | 51.4 |
| | #A | 7.2 | 6 | 53.7 |
| KDR5Δ1N-Fc | #1 | 11.7 | 16.2 | 55.6 |
| | #2 | 11.6 | 15.8 | 46.9 |
| | #3 | 7.9 | 34.3 | 70.3 |
| KDR2N-Fc | #1 | 0 | 2.6 | 27.6 |
| | #2 | 1.4 | 0.5 | 36.2 |

All of the antisera of three mice immunized with KDR-5Δ1N-Fc showed 50% or more of the binding inhibition activity by 100 times dilution, and the antiserum of one of the three animals showed the strongest binding inhibition activity of 34.3% by 1,000 times dilution. Antisera of three and two mice immunized with KDR-7N-Fc and KDR-5N-Fc, respectively, showed 50% or more of the binding inhibition activity by 100 times dilution. Accordingly, it was shown that the KDR-5Δ1N-Fc which does not contain the first Ig-like domain having the strongest binding inhibition activity and strong immunogenicity is suitable as the immunogen.

9. Screening of hybridoma by [125I]VEGF-KDR binding inhibition assay

When hybridomas were prepared from one mouse immunized with KDR-5N-Fc and screened by the [125I]VEGF-KDR binding inhibition assay described in 8 using the thus obtained culture supernatants of about 672 wells, 7 clones of hybridomas producing monoclonal antibody showing 90.1, 66.7, 59.0, 85.7, 86.8, 78.0 and 91.2% of the binding inhibition activity in culture supernatants were obtained and named KM1991 to KM1997, respectively (Table 4).

10. Epitope analysis of monoclonal antibodies KM1991 to KM1997

Specificity of the anti-human VEGF receptor KDR monoclonal antibodies described in 9 was confirmed by the enzyme immunoassay described in 3 using 5 μg/ml of purified antibodies.

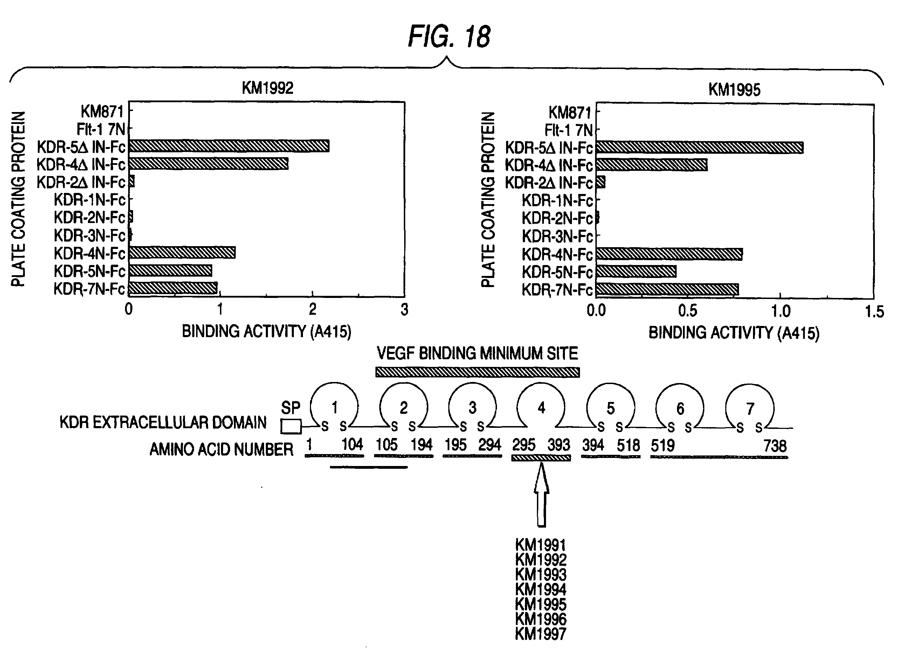

Typical results and summarized results are shown in Fig. 18 and Fig. 17, respectively. All of these 7 monoclonal antibodies represented by KM1992 and KM1995 reacted with the fourth Ig-like domain (corresponds to 295th to 393rd amino acids). Thus, it was shown that the fourth Ig-like domain (corresponds to 295th to 393rd amino acids) from the N-terminal site of KDR is particularly important for binding with VEGF. Particularly, the fact that KM1991, KM1992, KM1993, KM1994 and KM1995 having the activity to inhibit self-phosphorylation of VEGF receptor KDR described in 13 or the activity to inhibit growth of VEGF-dependent vascular endothelial cells described in 14 indicated that there are neutralizing monoclonal antibodies which inhibit biological activity of KDR. While a neutralizing monoclonal antibody capable of inhibiting biological activities of KDR was not able to obtain from a total of 74 clones of anti-human VEGF receptor KDR monoclonal antibodies obtained in 6, neutralizing monoclonal antibodies were obtained in 9, so that it was revealed that the KDR-5Δ1N-Fc which does not contain the first Ig-like domain having strong immunogenicity is suitable as the immunogen and that the [125I]VEGF-KDR binding inhibition assay is suitable as a hybridoma screening system.

In order to determine antibody class of the monoclonal antibodies, enzyme immunoassay was carried out using a subclass typing kit (manufactured by Zymed). The results are shown in the following Table 6.

Table 6

| Antibody class | KM Number |
|---|---|
| IgG1 | 1665, 1666, 1668, 1768, 1778-1780, 1825-1829, 1831, 1838, 1853-1858, 1862, 1863, 1865, 1943-1950, 1965, 1967, 1968, 1971-1975, 1987-1989, 1992, 1994 |
| IgG2a | 1830, 1859-1861, 1864, 1966, 1969, 1970 |
| IgG2b | 1993, 1995 |
| IgG3 | |
| IgM | 1659, 1942 |
| IgA | 1664 |
| IgE | 1991, 1996, 1997 |

All of the monoclonal antibodies established by the present invention were IgG class, excluding KM1659 and KM1942 as IgM, KM1664 as IgA and KM1991, KM1996 and KM1997 as IgE.

Industrial Applicability

According to the present invention, solid tumors, chronic rheumatoid arthritis, diabetic retinopathy, retinopathy of prematurity, psoriasis and the like can be treated more effectively by a medicament comprising a combination of a monoclonal antibody capable of binding specifically to human VEGF receptor Flt-1 with a monoclonal antibody capable of binding specifically to human VEGF receptor KDR. The medicament is useful for the diagnosis or treatment of diseases in which their morbid states progress by abnormal angiogenesis, such as proliferation or metastasis of solid tumors, arthritis in chronic rheumatoid arthritis, diabetic retinopathy, retinopathy of prematurity, psoriasis, and the like.

Free Text of Sequence Listings

SEQ ID NO:1-Explanation of artificial sequence: Synthetic DNA
SEQ ID NO:2-Explanation of artificial sequence: Synthetic DNA
SEQ ID NO:3-Explanation of artificial sequence: Synthetic DNA
SEQ ID NO:4-Explanation of artificial sequence: Synthetic DNA
SEQ ID NO:5-Explanation of artificial sequence: Synthetic DNA
SEQ ID NO:6-Explanation of artificial sequence: Synthetic DNA
SEQ ID NO:7-Explanation of artificial sequence: Synthetic DNA
SEQ ID NO:8-Explanation of artificial sequence: Synthetic DNA
SEQ ID NO:9-Explanation of artificial sequence: Synthetic DNA
SEQ ID NO:10-Explanation of artificial sequence: Synthetic DNA
SEQ ID NO:11-Explanation of artificial sequence: Synthetic DNA
SEQ ID NO:12-Explanation of artificial sequence: Synthetic DNA
SEQ ID NO:13-Explanation of artificial sequence: Synthetic DNA
SEQ ID NO:14-Explanation of artificial sequence: Synthetic DNA
SEQ ID NO:15-Explanation of artificial sequence: Synthetic DNA
SEQ ID NO:16-Explanation of artificial sequence: Synthetic DNA
SEQ ID NO:17-Explanation of artificial sequence: Synthetic DNA
SEQ ID NO:18-Explanation of artificial sequence: Synthetic DNA
SEQ ID NO:19-Explanation of artificial sequence: Synthetic DNA

SEQ ID NO:20-Explanation of artificial sequence: Synthetic DNA

SEQ ID NO:21-Explanation of artificial sequence: Synthetic DNA

SEQ ID NO:22-Explanation of artificial sequence: Synthetic DNA

SEQ ID NO:23-Explanation of artificial sequence: Synthetic DNA

SEQ ID NO:24-Explanation of artificial sequence: Synthetic DNA

SEQ ID NO:25-Explanation of artificial sequence: Synthetic DNA

SEQ ID NO:26-Explanation of artificial sequence: Synthetic DNA

SEQ ID NO:27-Explanation of artificial sequence: Synthetic DNA

SEQ ID NO:28-Explanation of artificial sequence: Synthetic DNA

SEQ ID NO:29-Explanation of artificial sequence: Synthetic DNA

SEQ ID NO:30-Explanation of artificial sequence: Synthetic DNA

SEQ ID NO:31-Explanation of artificial sequence: Synthetic DNA

SEQ ID NO:32-Explanation of artificial sequence: Synthetic DNA

SEQ ID NO:33-Explanation of artificial sequence: Synthetic DNA

**Claims**

1. A medicament comprising a combination of a substance which inhibits signal transduction mediated by human VEGF receptor Flt-1 with a substance which inhibits signal transduction mediated by human VEGF receptor KDR.

2. A VEGF activity inhibitor comprising a combination of a substance which inhibits signal transduction mediated by human VEGF receptor Flt-1 with a substance which inhibits signal transduction mediated by human VEGF receptor KDR.

3. An angiogenesis inhibitor comprising a combination of a substance which inhibits signal transduction mediated by human VEGF receptor Flt-1 with a substance which inhibits signal transduction mediated by human VEGF receptor KDR.

4. A therapeutic agent for a disease in which the morbid states progress by abnormal angiogenesis, comprising a combination of a substance which inhibits signal transduction mediated by human VEGF receptor Flt-1 with a substance which inhibits signal transduction mediated by human VEGF receptor KDR.

5. The therapeutic agent according to claim 4, wherein the disease in which the morbid states progress by abnormal angiogenesis is proliferation or metastasis of a solid tumor, arthritis in rheumatoid arthritis, diabetic retinopathy, retinopathy of prematurity, or psoriasis.

6. An agent according to claims 1 to 5, wherein the substance which inhibits signal transduction mediated by human VEGF receptor Flt-1 is a substance which inhibits binding of VEGF to the Flt-1 receptor or a substance which inhibits signal transduction from the Flt-1 receptor.

7. The agent according to claim 6, wherein the substance which inhibits binding of VEGF to the Flt-1 receptor is selected from an anti-human VEGF receptor Flt-1 monoclonal antibody and a fragment of the antibody.

8. The agent according to claim 7, wherein the anti-human VEGF receptor Flt-1 monoclonal antibody is a monoclonal antibody belonging to the mouse IgG2b subclass produced by a hybridoma KM1750 (FERM BP-5700) or a monoclonal antibody belonging to the mouse IgG1 subclass produced by a hybridoma KM1732 (FERM BP-5698).

9. The agent according to claim 6, wherein the substance which inhibits signal transduction from the Flt-1 receptor is selected from a substance having Flt-1 tyrosine kinase inhibition activity and a substance having p38 inhibition activity.

10. An agent according to claims 1 to 5, wherein the substance which inhibits signal transduction mediated by human VEGF receptor KDR is a substance which inhibits binding of VEGF to the KDR receptor or a substance which inhibits signal transduction from the KDR receptor.

11. The agent according to claim 10, wherein the substance which inhibits binding of VEGF to the KDR receptor is selected from an anti-human VEGF receptor KDR monoclonal antibody and a fragment of the antibody.

**12.** The agent according to claim 11, wherein the anti-human VEGF receptor KDR monoclonal antibody is a monoclonal antibody belonging to the mouse IgG1 subclass produced by a hybridoma KM1992 (FERM BP-6217) or a monoclonal antibody belonging to the mouse IgG2b subclass produced by a hybridoma KM1995 (FERM BP-6218).

**13.** The agent according to claim 10, wherein the substance which inhibits signal transduction from KDR receptor is selected from a substance having KDR tyrosine kinase inhibition activity and a substance having ERK inhibition activity.

**14.** A medicament comprising a human VEGF receptor Flt-1 antagonist and a human VEGF receptor KDR antagonist.

*FIG. 1*

EP 1 086 705 A1

| | | | | | |
|---|---|---|---|---|---|
| VEGF | − | + | + | + | + |
| ANTI-Flt-1 MONOCLONAL ANTIBODY | − | − | + | − | + |
| ANTI-KDR MONOCLONAL ANTIBODY | − | − | − | + | + |

*FIG. 2*

VEGF: 10ng/ml

VEGF: 10ng/ml

| | | | | |
|---|---|---|---|---|
| VEGF | − | + | + | + | + |
| ANTI-Flt-1 MONOCLONAL ANTIBODY | − | − | + | − | + |
| ANTI-KDR MONOCLONAL ANTIBODY | − | − | − | + | + |

FIG. 3

EP 1 086 705 A1

| | | | | |
|---|---|---|---|---|
| flt-1 | | | | |
| KDR | | | | |
| ets-1 | | | | |
| MMP-1 | | | | |
| GAPDH | | | | |

| | | | | | |
|---|---|---|---|---|---|
| VEGF | − | + | + | + | + |
| ANTI-Flt-1 MONOCLONAL ANTIBODY | − | − | + | − | + |
| ANTI-KDR MONOCLONAL ANTIBODY | − | − | − | + | + |

Fig. 4

FIG. 5

| | | | | | |
|---|---|---|---|---|---|
| VEGF 10ng/ml | − | + | + | + | + |
| ANTI-Flt-1 MONOCLONAL ANTIBODY | − | − | + | − | + |
| ANTI-KDR MONOCLONAL ANTIBODY | − | − | − | + | + |

EP 1 086 705 A1

FIG. 6

# FIG. 7

POLYHEDRAL PROMOTER

EcoRI
NotI

pVL1393

Ap

EcoRI
TaqI

HUMAN VEGF RECEPTOR Flt-1 cDNA

EcoRI
TaqI

EcoRI-TaqI
FRAGMENT (1263 bp)

TaqI-NotI ADAPTER

SYNTHETIC DNA: SEQ ID NOS: 5 AND 6

EcoRI
NotI

POLYHEDRAL PROMOTER
EcoRI

Flt 3N

pVL1393/Flt 3N

TaqI
NotI

Ap

# FIG. 8

# FIG. 9

kDa

208 →
144 →
87 →
44.1 →
32.7 →
17.7 →

1  2  3

LANE

1. MOLECULAR WEIGHT MARKER
2. Flt-1 (3N)
3. Flt-1 (7N)

## FIG. 10

EP 1 086 705 A1

FIG. 11

*FIG. 12*

EP 1 086 705 A1

SIGNAL SEQUENCE

EXTRACELLULAR DOMAIN

TRANSMEMBRANE DOMAIN

TYROSINE KINASE DOMAIN

HUMAN KDR
1 2 3 4 5 6 7

KDR-7N-Fc (971 a.a.)
-19 1
1 2 3 4 5 6 7
LINKER #1 + Fc (6 a.a + 227 a.a)
738

KDR-5N-Fc (751 a.a.)
-19 1
1 2 3 4 5
LINKER #1 + Fc (6 a.a + 227 a.a)
518

KDR-4N-Fc (622 a.a.)
-19 1
1 2 3 4
LINKER #2 + Fc (2 a.a + 227 a.a)
393

KDR-3N-Fc (527 a.a.)
-19 1
1 2 3
LINKER #1 + Fc (6 a.a + 227 a.a)
294

KDR-2N-Fc (427 a.a.)
-19 1
1 2
LINKER #1 + Fc (6 a.a + 227 a.a)
194

KDR-1N-Fc (337 a.a.)
-19 1
1
LINKER #1 + Fc (6 a.a + 227 a.a)
104

KDR-5 Δ1N-Fc (679 a.a.)
-19 1 (Δ31-102)
2 3 4 5
LINKER #1 + Fc (6 a.a + 227 a.a)
518

KDR-4 Δ1N-Fc (550 a.a.)
-19 1 (Δ31-102)
2 3 4
LINKER #2 + Fc (2 a.a + 227 a.a)
393

KDR-2 Δ1N-Fc (355 a.a.)
-19 1 (Δ31-102) 194
2
LINKER #1 + Fc (6 a.a + 227 a.a)

*FIG. 13*

# FIG. 14

REDUCING CONDITIONS
2μg PROTEIN/LANE

*FIG. 15A*

COMPETITIVE INHIBITION ACTIVITY OF SOLUBLE KDR DERIVATIVE

Legend:
- KDR-2N-Fc
- KDR-3N-Fc
- KDR-4N-Fc
- KDR-5N-Fc
- KDR-7N-Fc
- KDR-2Δ1N-Fc
- KDR-4Δ1N-Fc
- KDR-5Δ1N-Fc

*FIG. 15B*

VEGF BINDING ACTIVITY OF SOLUBLE KDR DERIVATIVE

FIG. 16

(CONT.)

(FIG. 16 CONTINUED)

FIG. 17

VEGF BINDING MINIMUM SITE

KDR EXTRACELLULAR DOMAIN

SP  1  2  3  4  5  6  7

AMINO ACID NUMBER  1  104  105  194  195  294  295  393  394  518  519  738

SCREENING OF HYBRIDOMA:
BINDING ELISA

| KM1659 | KM1860# | KM1987# | KM1778 | KM1971 | KM1854 | KM1780 | KM1665 |
| KM1664 | KM1861# | KM1988# | KM1779 | KM1858# | KM1968 | KM1965 | KM1666 |
| KM1668# | KM1862# | KM1989# | KM1830 | | KM1974 | KM1966 | |
| KM1768# | KM1863# | | KM1855 | | | KM1967 | |
| KM1825# | KM1864# | | KM1973 | | | KM1969 | |
| KM1826# | KM1865# | | | | | KM1970 | |
| KM1827# | KM1933# | | | | | KM1972 | |
| KM1828# | KM1942# | | | | | KM1975 | |
| KM1829# | KM1943# |
| KM1831# | KM1944# |
| KM1835# | KM1945# |
| KM1837# | KM1946# |
| KM1853# | KM1947# |
| KM1856# | KM1948# |
| KM1857# | KM1949# |
| KM1859# | KM1950# |

KM1832#
KM1833#
KM1834#
KM1836#
KM1838#
KM1982#

#KDR REACTIVITY ON CELL

VEGF-SOLUBLE KDR BINDING INHIBITION TEST

KM1991#
KM1992#
KM1993#
KM1994#
KM1995#
KM1996#
KM1997#

EP 1 086 705 A1

## FIG. 18

# INTERNATIONAL SEARCH REPORT

| International application No. |
| --- |
| PCT/JP99/02660 |

| A. CLASSIFICATION OF SUBJECT MATTER |
| --- |
| Int.Cl⁶ A61K45/06, 39/395 // A61K31/35, 31/40, 31/44 |
| According to International Patent Classification (IPC) or to both national classification and IPC |

| B. FIELDS SEARCHED |
| --- |
| Minimum documentation searched (classification system followed by classification symbols) |
| Int.Cl⁶ A61K45/06, 39/395 // A61K31/35, 31/40, 31/44 |
| Documentation searched other than minimum documentation to the extent that such documents are included in the fields searched |
| Electronic data base consulted during the international search (name of data base and, where practicable, search terms used) |
| CAPLUS (STN), REGISTRY (STN) |

## C. DOCUMENTS CONSIDERED TO BE RELEVANT

| Category* | Citation of document, with indication, where appropriate, of the relevant passages | Relevant to claim No. |
| --- | --- | --- |
| X | WO, 95/21868, A1 (IMCLONE SYSTEMS INCORPORATED), 17 August, 1995 (17. 08. 95), Reference as a whole (particularly Claim 4 ; page 12, line 18 to page 26, line 12) & US, 5840301, A & US, 5861499, A & US, 5874542, A & EP, 741748, A1 | 1-8, 10-12, 14 |
| Y | WO, 97/15662, A2 (RIBOZYME PHARMACEUTICALS), 1 May, 1997 (01. 05. 97), Page 2, line 33 to page 3, line 5 ; page 5, line 23 to page 6, line 7 ; page 18, lines 6 to 25 (Family: none) | 1-14 |
| Y | WO, 97/34920, A1 (SUGEN, INC.), 25 September, 1997 (25. 09. 97), Page 33, lines 3 to 39, particularly Table II (Family: none) | 9, 13 |

| ☒ | Further documents are listed in the continuation of Box C. | ☐ | See patent family annex. |
| --- | --- | --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | |
| "O" document referring to an oral disclosure, use, exhibition or other means | "Y" document of particular relevance; the claimed invention cannot be considered to involve an inventive step when the document is combined with one or more other such documents, such combination being obvious to a person skilled in the art |
| "P" document published prior to the international filing date but later than the priority date claimed | "&" document member of the same patent family |

| Date of the actual completion of the international search | Date of mailing of the international search report |
| --- | --- |
| 27 July, 1999 (27. 07. 99) | 10 August, 1999 (10. 08. 99) |

| Name and mailing address of the ISA/ | Authorized officer |
| --- | --- |
| Japanese Patent Office | |
| Facsimile No. | Telephone No. |

Form PCT/ISA/210 (second sheet) (July 1992)

## INTERNATIONAL SEARCH REPORT

| | International application No. |
|---|---|
| | PCT/JP99/02660 |

C (Continuation).  DOCUMENTS CONSIDERED TO BE RELEVANT

| Category* | Citation of document, with indication, where appropriate, of the relevant passages | Relevant to claim No. |
|---|---|---|
| Y | ROUSSEAU, Simon et al., p38 MAP kinase activation by vascular endothelial growth factor mediates actin reorganization and cell migration in human endothelial cells, Oncogene, 1997, Vol. 15, pp.2169-2177 Reference as a whole | 9, 13 |

Form PCT/ISA/210 (continuation of second sheet) (July 1992)